# EUROPEAN PATENT APPLICATION

(11) **EP 4 388 881 A1**
(43) Date of publication of application: **26.06.2024**
(21) Application number: 23165813.9
(22) Date of filing: 31.03.2023
(51) Int. Cl.: A22C 17/00, A23N 15/02, A01F 11/06

(54) **FOOD IMAGING AND PROCESSING SYSTEMS AND METHODS**

(30) Priority: 21.12.2022 US 202263476526 P; 15.02.2023 US 202318169331
(71) Applicant: Proex Food, LLC, Menomonee Falls, WI 53051 (US)
(72) Inventor: GHADIRI, Daniel, Chicago, 60613 (US)
(74) Representative: Håmsø Patentbyrå AS

(57) **Abstract**

A food processing system for processing a food product, includes imaging system for generating an image of the food product and a control system. The control system is configured to process the image of the food product and determine a determine a product region of the image related to the food product. A robot is configured to move the food product into a processing system. The processing system is configured to cut the food product and dispense a processed food product that corresponds to the desired product region.

## Description

### CROSS-REFERENCE TO RELATED APPLICATION

The present application claims priority of U.S. Provisional Patent Application No. 63/476,526, filed December 21, 2022.

### FIELD

The present disclosure relates to food imaging and processing systems and methods.

### BACKGROUND

Food products, such as produce, are harvested and taken to facilities in which the food products are processed for consumption. Some facilities process the food products for further processing at other facilities and other facilities process the food products for sale in grocery stores and convenience stores. The facilities process large quantities of food products. In general, produce processing facilities operations focus on cleaning, processing, and packaging produce for sale in grocery or convenience stores. Generally, this done manually by workers, with relatively large amounts of waste, consistency of finished product can vary greatly.

The following U.S. Patents and U.S. Patent Application Publications are referred to for relevant background information.

U.S. Patent No. 10,405,813 discloses systems and methods for obtaining a panoramic images.

U.S. Patent No. 10,539,516 discloses systems for generating frame data based on the fan-shaped spreading of an X-ray beam and the differences in position in a height direction between the tomographic planes from a detection surface.

U.S. Patent Application Publication No. 2012/0307013 discloses food processing apparatuses for detecting and cutting tough tissues of food items.

U.S. Patent Application Publication No. 2022/0256869 discloses methods for processing and grading food articles including x-raying the food articles.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In certain examples, a food processing system for processing a food product includes an imaging system for generating an image of the food product and a control system configured to process the image of the food product and determine a product region related to the food product. A robot is configured to move the food product into a processing system, and the processing system is configured to cut the food product and dispense a processed food product that corresponds to the product region.

In certain examples, a food processing system for processing a plurality of food products includes an imaging system having an x-ray for generating an image of each food product of the plurality of food product and a control system configured to process the image of each food product and determine a cutline for the image and a corresponding cutline plane for each food product along which the food product is cut. A conveyor is configured to convey each food product through the food processing system and a robot is configured to move each food product from the conveyor into a processing system. The processing system includes a processing conveyor onto which each food product is received from the robot. A blade is configured to cut each food product based on the respective cutline plane for each food product and the processing conveyor conveys the food product past the one or more blades such that each food product is a processed food product.

In certain examples, a food processing system for individually processing ears of corn includes an imaging system having an x-ray for generating an image of each ear of corn. A control system is configured to receive the image for each ear of corn from the imaging section and process the image to identify defects in the ear of corn and determine a product region of the ear of corn based on the identified defects. A conveyor is configured to convey each ear of corn through the food processing system. A robot is configured to move each ear of corn from the conveyor into a processing system. The processing system includes a processing conveyor onto which each ear of corn is received from the robot and a blade configured to cut each ear of corn based on the product region determined for each ear of corn such that each ear of corn is a processed ear of corn that corresponds to the product region.

In certain examples a method for processing a food product includes the steps of generating an image of a food product by means of an imaging system; processing the image of the food product and determining a product region related to the food product by means of a control system; moving the food product into a processing system by means of a robot; and by means of the processing system, cutting the food product and dispensing a processed food product that corresponds to the product region.

Various other features, objects, and advantages will be made apparent from the following description taken together with the drawings.

### BRIEF DESCRIPTION OF THE DRAWINGS

The patent or application file contains at least one drawing executed in color. Copies of this patent or patent application publication with color drawing(s) will be provided by the Office upon request and payment of the necessary fee. The present disclosure is described with reference to the following Figures. The same numbers are used throughout the Figures to reference like features and like components.
Fig. 1 is a perspective view of an example food processing system according to the present disclosure.
Fig. 2 is a perspective view of an example input section according to the present disclosure.
Fig. 3 is a top-down plan view of the input section of Fig. 2 without corn loaded thereon.
Fig. 4 is a view like Fig. 3 with corn loaded onto a first example shaker table module.
Fig. 5 is a view like Fig. 3 with the corn moved onto a second example shaker table module.
Fig. 6 is a view like Fig. 3 with the corn moved onto a third example shaker table module.
Fig. 7 is a view like Fig. 3 with the corn moved onto a fourth example shaker table module and a conveyor of an imaging section.
Fig. 8 is a perspective view of an example imaging section according to the present disclosure.
Fig. 9 is top-down plan view of the imaging section of Fig. 8.
Fig. 10 is a cross-sectional view of the imaging section along line 10-10 on Fig. 9.
Fig. 11 is a schematic diagram of an example control system according to the present disclosure.
Fig. 12 is an example processed image depicting an ear of corn. The darker regions of the image are kernels and the cob of the corn while the lighter regions of the image are undesirable components of the corn such as the husk.
Fig. 13 is a schematic top-down plan view of the conveyor in an imaging section of the system according to the present disclosure.
Fig. 14 depicts an image of a corn with normal corn kernels and a tip area of the cob devoid of kernels.
Fig. 15 depicts an image of a corn with normal corn kernels and a body area of the cob devoid of kernels.
Fig. 16 depicts an image of a corn with normal corn kernels and a butt area of the cod devoid of kernels.
Fig. 17 depicts an image of a corn that have been mechanically damaged with large cuts or missing section due to the harvesting process.
Figs. 18-19 depict images of corn with normal corn kernels and kernels that are dented or dimpled due to improper growth.
Figs. 20-21 depict images of corn with normal corn kernels and kernels that have been damaged by insects.
Fig. 22 depicts an image of a corn with normal corn kernels and smut at the tip of the cob.
Fig. 23 depicts an image of a corn with normal corn kernels and decayed kernels in the body section of the corn.
Fig. 24 depicts an image of a corn with normal corn kernels and kernels that are dehydrated near the tip of the cob.
Fig. 25 depicts an image of a corn with normal corn kernels and kernels that are dehydrated along a section of the body.
Figs. 26-28 depicts images of corn having immature, not fully developed kernels.
Figs. 29-30 depict images of corn with normal corn kernels and kernels that have been damaged by birds.
Fig. 31 depicts images corn having short and inadequate length.
Fig. 32 depicts an image of corn with normal kernels and an area of the corn with dehydrated kernels and tip blanking.
Fig. 33 depicts an x-ray image corresponding to the image of Fig. 32.
Fig. 34 depicts a corn with normal kernels and an area of the corn with body dehydration and body blanking.
Fig. 35 depicts an x-ray image corresponding to the image of Fig. 34.
Fig. 36 depicts a corn with normal kernels and devoid of defects.
Fig. 37 depicts an x-ray image corresponding to the image of Fig. 36.
Fig. 38 is a perspective view of an example sorting section according to the present disclosure.
Fig. 39 is an enlarged perspective view of an example sorting section according to the present disclosure with corn on a conveyor of an imaging section.
Fig. 40 is another enlarged perspective view of an example sorting section according to the present disclosure with corn on a conveyor of an imaging section and corn on a conveyor of a processing section.
Fig. 41 is a schematic view of a processed corn with ends cut off.
Fig. 42 is a top-down plan view of the processing section depicted in Fig. 38.
Fig. 43 is an end view of the processing section depicted in Fig. 42.
Fig. 44 is an enlarged view of another processing section according to the present disclosure.
Fig. 45 is another example food processing system according to the present disclosure.
Fig. 46 depicts an example process flow according to the present disclosure.

### DETAILED DESCRIPTION

The present inventors have recognized there is a need in the food product (e.g., produce such as ears of corn) processing industry to identify the quality of the products, the characteristics of the products, and/or process the products into salable products all while increasing the number of products that are can be received and processed in a given processing facility. In addition, the number of food products received by some processing facilities is increasing dramatically as facilities are consolidated. As such, the present inventor has endeavored to develop the systems described herein below that image and process the products to thereby increase the efficiency and effectiveness of the processing facility (as will be described in greater detail hereinbelow). The systems can also decrease the amount of labor needed for quality control in processing facilities, while also improving accuracy and efficiency of quality control.

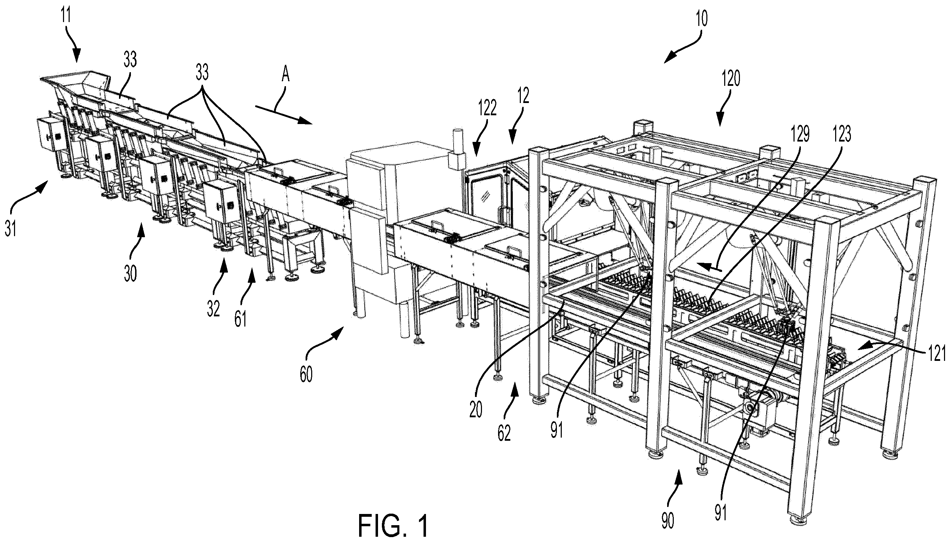

Fig. 1 depicts an example food product processing system 10 of the present disclosure. The system 10 includes an upstream first end 11 and a downstream second end 12. The system 10 includes one or more sections (described further herein below) positioned between the ends 11, 12. The system 10 also includes one or more conveyors 20 that extend between the ends 11, 12 to convey food products, such as produce, from the first end 11 through the various section (described further herein) and to the second end 12. The first direction of conveyance of the products through the system 10 is depicted by arrow A (hereafter the "downstream direction"). In one example, the conveyor 20 is an endless belt conveyor with a belt on which the products are carried. Note that in other examples the conveyor 20 could convey other devices, e.g., chains, plates, hooks, and/or troughs, through the system 10. As noted above, the system 10 is configured to convey food products therethrough The type of food products can vary such as produce and meat. In certain examples, the system 10 is capable of receiving and processing produce such as ears of corn, romaine lettuce, leafy greens, artichokes, cabbage, asparagus, celery, tomatoes, potatoes, apples, stone fruits, peppers, and pomegranate. Note that while the below-described examples of the system 10 reference ear(s) of corn (also referred to as "com" for simplicity), the example systems 10 are not limited to corn such that the example systems 10 and the features or components of the system 10 can be utilized with any other food products that can be processed with the sections, components, and/or features of the example systems 10 described herein.

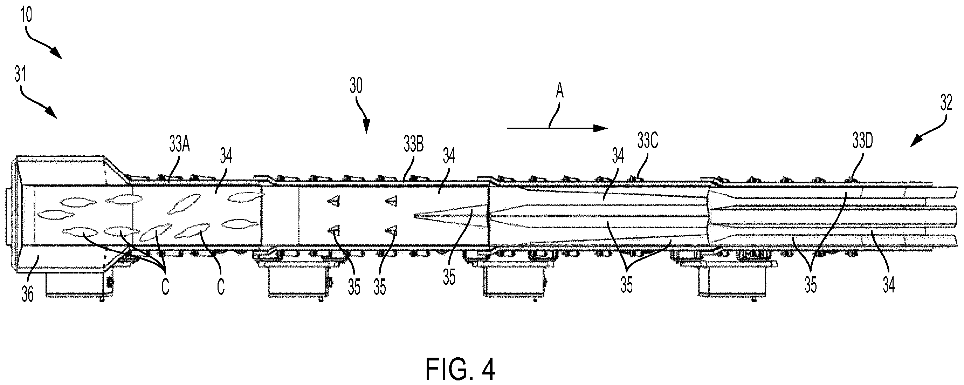

The system 10 includes (starting in order from the first end 11 to the second end 12) an input section 30 that receives corn C (see Fig. 4) from upstream infeed equipment or machines (not depicted; e.g., hopper) and dispenses the corn C to a downstream imaging section 60. The imaging section 60 is configured to generate an image of each corn C. The corn C is conveyed by one or more conveyors 20 downstream to a sorting section 90 that is configured to sort the corn C based on the generated image. The sorted corn C is processed by a processing section 120 such that the corn C can be subsequently packaged and shipped. The sections 30, 60, 90, 120 of the system 10 are described in greater detail hereinbelow.

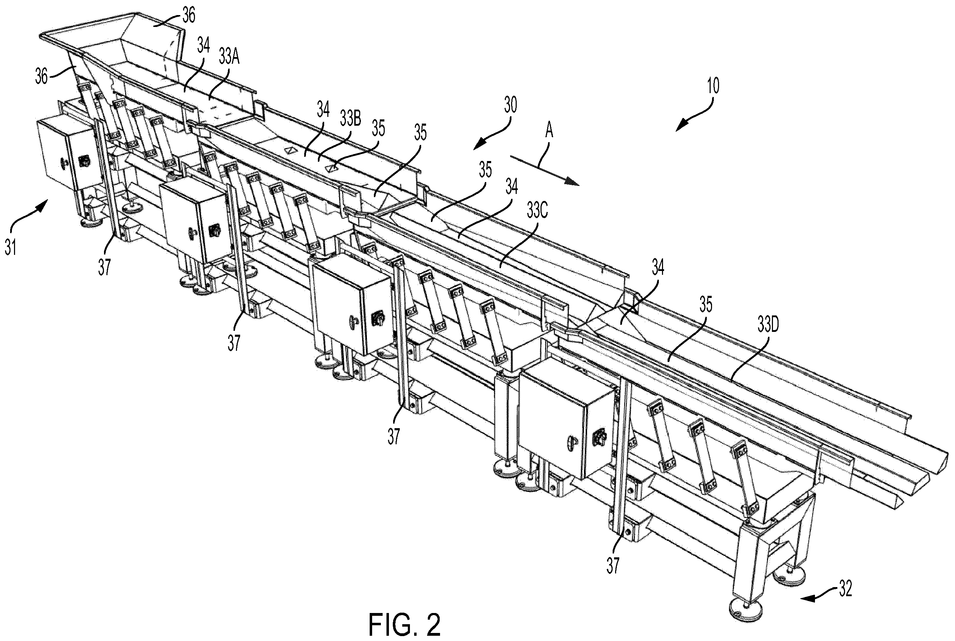

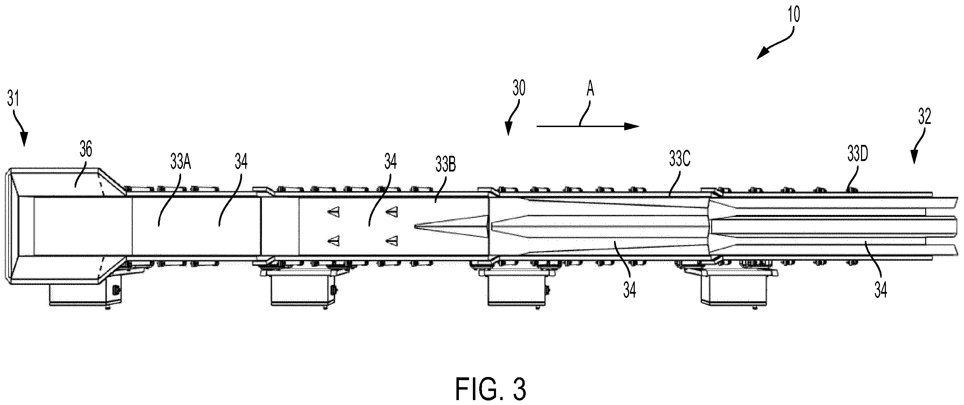

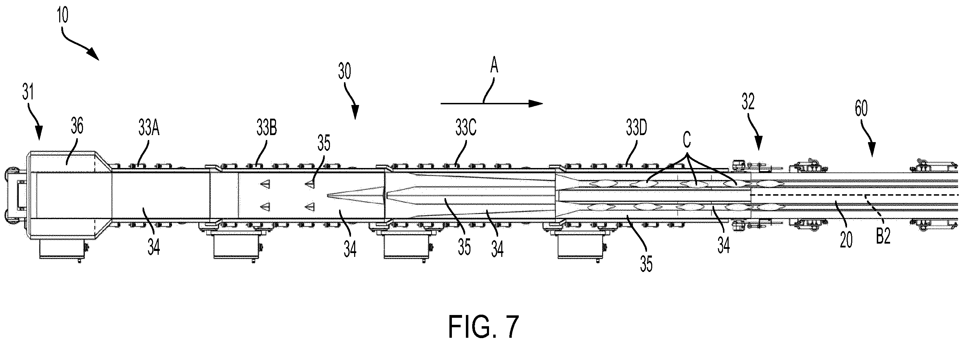

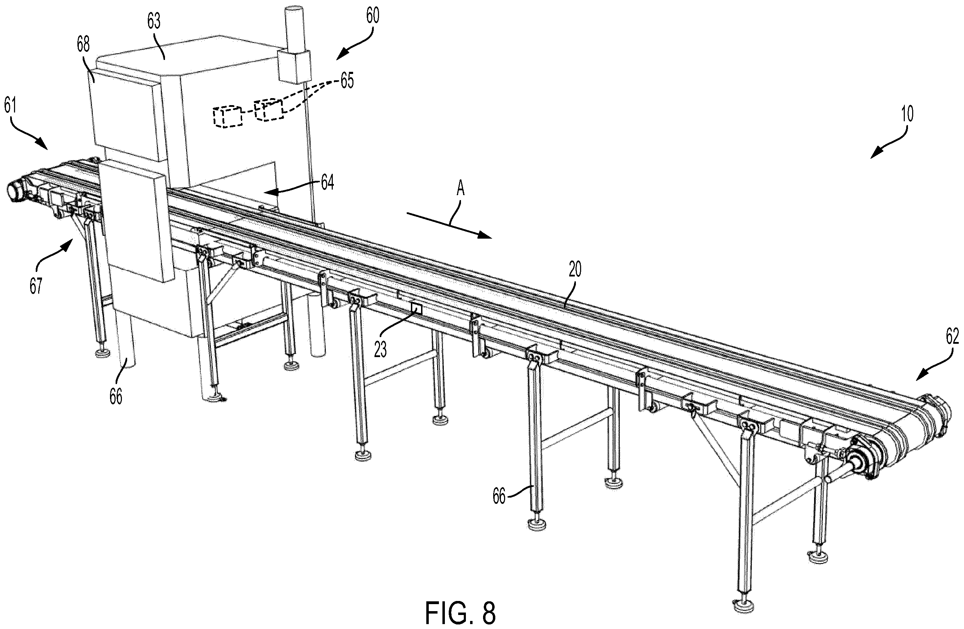

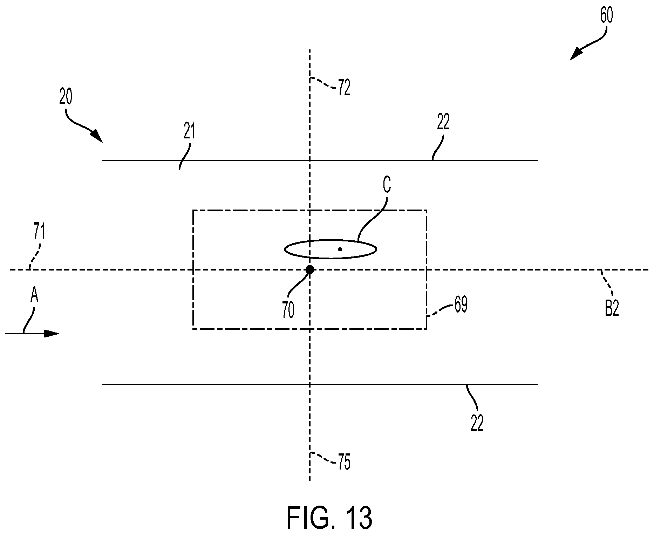

Referring to Figs. 2-7 depict an example input section 30 in more detail. The input section 30 includes an upstream first end 31 that receives the corn and an opposite downstream second end 32 that dispenses the corn to the imaging section 60 (Fig. 8). A plurality of shaker table modules 33 are positioned between the end 31, 32, and the shaker table modules 33 are configured to move the corn C in the downstream direction (arrow A) as an even flow of corn C such that the corn C dispense in one or more rows and are orientated lengthwise with a longitudinal corn axis of the corn C (axis extends between the tip end and the tail end of the corn C) generally extend in the downstream direction (see Fig. 13) and along (e.g., parallel with) the longitudinal belt axis B2 (see Fig. 13) and spaced apart from each other (see Fig. 7 in which the corn C are in two rows and adjacent ends of the corn C are spaced apart by approximately 1.0 inches). The orientation and spacing between the corn C is advantageous for imaging of the corn C in the image section 60 (described in greater detail below).

The number of shaker table modules 33 can vary, and in the example depicted in Figs. 2-4, four shaker table modules 33 are depicted. Each shaker table module 33 includes a frame 37 that vertically supports a table or slide 34 on which the corn is moved. The slide 34 is vertically downwardly tilted. Each module 33 includes an actuator (e.g., motor with a vibrator) that causes the slide 34 to vibrate or shake. As such, the slide 34 causes the corn C positioned thereon to shake and slide in the downstream direction (see arrow A) to the next downstream module 33 and eventually to the imaging section 60. As the corn C is moved in the downstream direction (see arrow A), various orientation components 35 extending from the slide, such as angled plates, bumpers, guides, grooves, and walls, align the corn lengthwise.

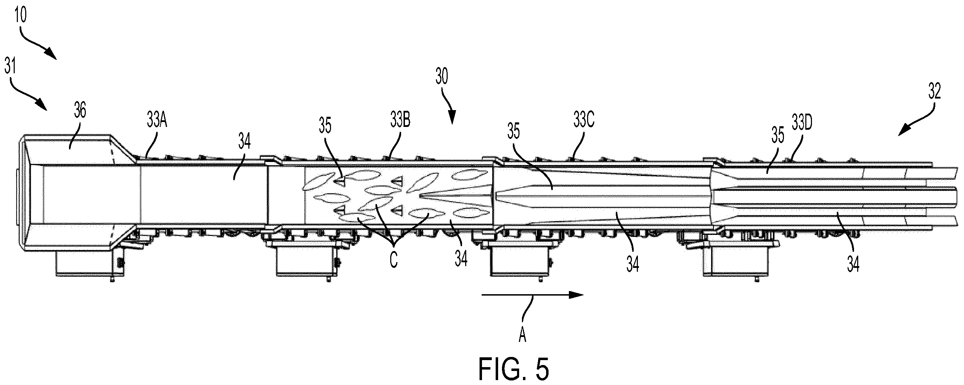

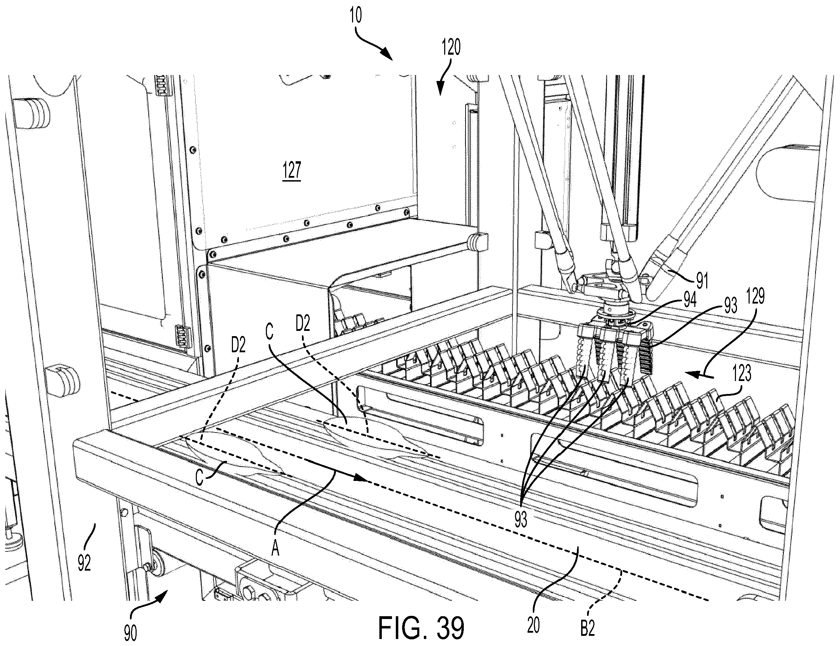

An example operational sequence of orientating the corn as depicted in Figs. 4-7. Note the shaker table modules 33 depicted in these Figures are indicated as a first module 33A, a second module 33B, a third module 33C, and a fourth module 33D. In operation, the modules 33A-33D are placed into an operation condition (e.g., "ON") such that the slides 34 are shaking. The operator then loads the corn C onto the first module 33A as depicted in Fig. 4. Side panels 36 direct the corn C toward the middle of the module 33A and the sloped and shaking slide 34 causes the corn C to move in the downstream direction (arrow A). As the corn C are moved, the corn C begin to align lengthwise in the downstream direction (arrow A) such that the longitudinal corn axis D2 (see Fig. 39) of each corn C and generally extend along the longitudinal belt axis B2 (Figs. 7 and 114) . The corn C are dispensed from the first module 33A to the second module 33B (see Fig. 5) where the corn C are further shaken by the slide 34 of the second module 33B. Several orientation components 35 (e.g., triangularly shaped projections extending vertically away from the slide 34) cause the corn C to begin moving into rows of corn C as the shaking slide 34 of the second module 33B causes the corn C to move in the downstream direction (arrow A).

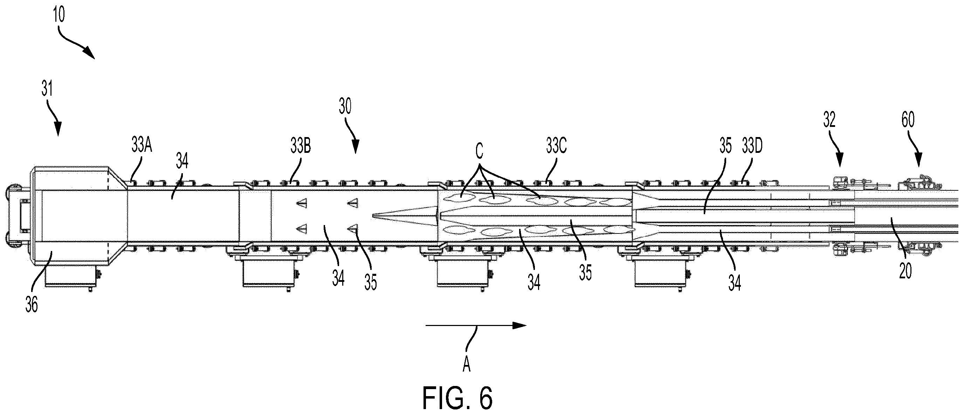

The corn C are dispensed from the second module 33B to the third module 33C (Fig. 6) where the corn C are further shaken by the slide 34 of the third module 33C. Several orientation components 35 (e.g., triangularly shaped projections elongated in the downstream direction) cause the corn C to move into the rows of corn C as the shaking slide 34 of the third module 33C causes the corn C to move in the downstream direction (arrow A).

The corn C are dispensed from the third module 33B to the fourth module 33D (Fig. 7) where the corn C are further shaken by the slide 34 of the fourth module 33D. Additional orientation components 35 (e.g., triangularly shaped projections elongated in the downstream direction) cause the corn C to move into and stay in rows of corn C as the shaking slide 34 of the third module 33D causes the corn C to move in the downstream direction (arrow A). In certain examples, the slide 34 of the third module 33C is shaken at a different speed or amplitude than the slide 34 of the fourth module 33D such that the corn C separate from each other in the downstream direction (arrow A) and thus the corn C are spaced apart from each other in one or more rows. In one non-limiting example, the corn C dispensing from the fourth module 33D are spaced apart from each by 1.0 inches.

In operation, as the conveyor 20 conveys the corn C that are received from the input section 30 and arranged in one or more rows, an imaging system 67 of the imaging section 60 generates an image (e.g., two-dimensional image (2D) or three-dimensional image (3D)) of the corn C and/or data corresponding to the corn C. As noted above, in the example system 10 depicted in Figs. 1-7 the corn C are dispensed onto a conveyor 20 such that the corn C are orientated in the lengthwise direction (see arrow A), organized into the two rows, and the corn C in each row are spaced apart from each other.

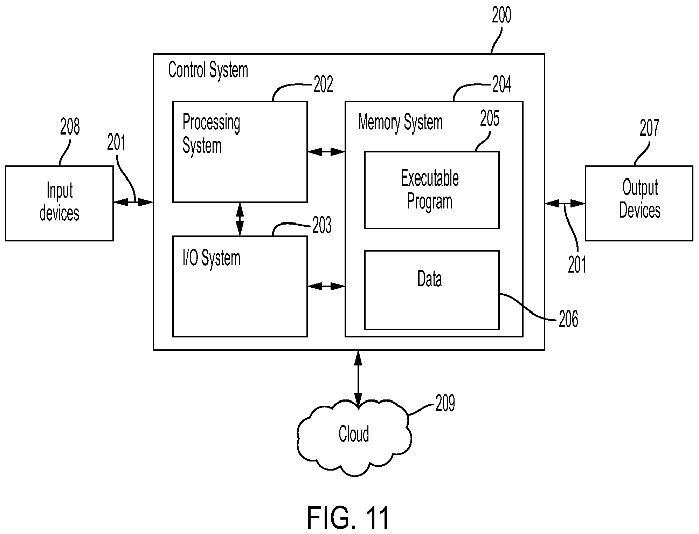

For the example system 10 depicted in Fig. 1, the imaging system 67 includes an imaging control system 68 (Fig. 8) that processes the images and outputs data and other signals to the control system 200 (Fig. 11) of the system 10 such that further operations of the system 10 can be controlled by the control system 200 based on the image and/or data. For instance, the image and data can be used for quality control purposes, sorting the corn C in the sorting section 90, and/or processing the corn C in the processing section 120 (described further herein). Note that in other examples, the image generated by the imaging system 67 is processed by the control system 200. Note that the imaging control system 68 can include any of the features or components described below with respect a control system 200 of the system 10 and vice versa. Further note that any of the features, processes, or method steps described with relation to the imaging control system 68 can be utilized with the control system 200 and vice versa.

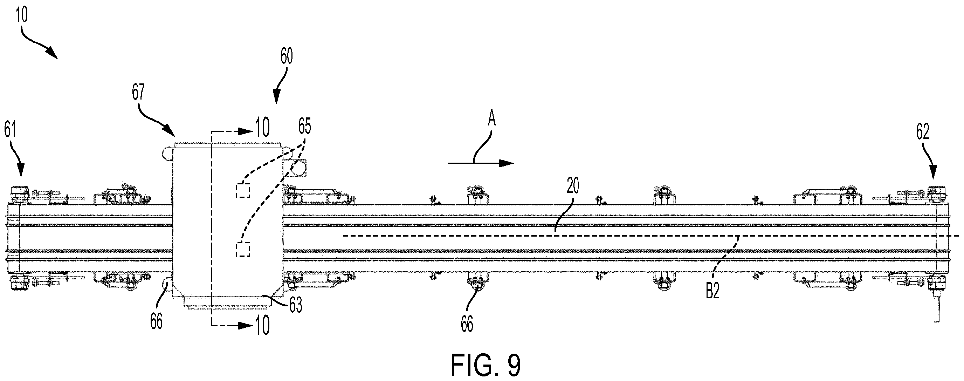

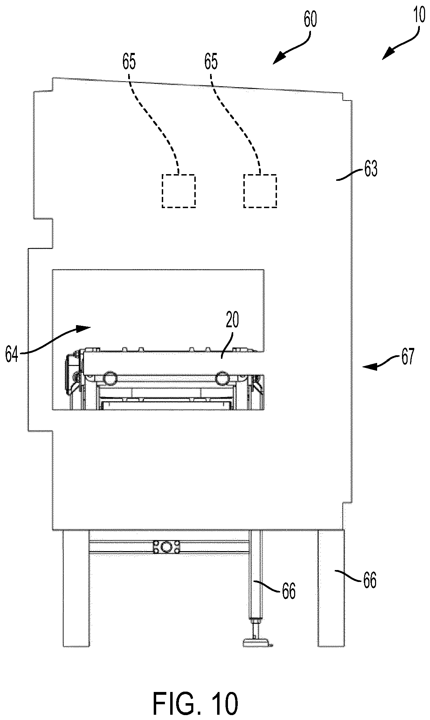

Referring now to Figs. 8-10, an example imaging section 60 is depicted in greater detail. The imaging section 60 includes an upstream first end 61 and an opposite second end 62. The conveyor 20 extends through the imaging section 60 and between the ends 61, 62. In general, the conveyor 20 is configured to receive the corn C from the input section 30 (as described above), convey the corn C through the imaging system 67 which generates an image of the corn C, and dispense the corn C to the sorting section 90 (described herein below). The imaging system 67 is configured to generate an image of each ear of corn C as the conveyor 20 continuously or intermittently conveys the corn in the downstream direction (arrow A) past an imaging device 65 (described in greater detail hereinbelow).

The imaging system 67 includes a housing 63 that houses and protects the imaging device 65. The housing 63 also prevents or reduces energy (e.g., x-rays) from leaking out of the imaging section 60 and further prevents water and dust/debris ingress. The housing 63 also defines a tunnel 64 through which the conveyor 20 extends. Note that the imaging section 60 includes a frame 66 that vertically supports the conveyor 20 and other components of the imaging section 60.

One or more one or more imaging devices 65 can be included with the imaging system 67. In one example, each imaging device 65 is for imaging the corn C in one of the rows of corn C (e.g., a first imaging device 65 images the corn C in the first row, a second imaging device 65 images the corn C in the second row, etc.). In other examples, an imaging device 65 is configured to image the corn C in more than one row (e.g., a first imaging device 65 images the corn C in two adjacent rows of corn, a second imaging device 65 images the corn C in two rows of corn).

The type of imaging device 65 utilized in the imaging section 60 can vary, and in one non-limiting example, the imaging device 65 is an x-ray. The x-ray image produced by the x-ray that is processed by the imaging control system 68 such that the image and/or data can be used for different quality control, sorting, robot operations, and/or processing the corn C, some of which are described herein below. In other examples, the imaging device 65 is a camera capable of imaging visible light wavelengths.

In examples where the imaging device 65 is an x-ray, the x-ray includes the imaging control system 68 that is in communication with one or more x-ray sources/generators and one or more x-ray receptors spaced apart from the x-ray sources (not depicted). The x-ray receptor can include a receptor array having a plurality of detection elements for detecting the radiation passing through the corn C and/or the conveyor 20 and generates an image and/or data corresponding the x-rays detected by the plurality of detection elements. In one example, the x-ray receptor is vertically below the conveyor 20 and vertically aligned with the x-ray generator (not depicted) that is positioned vertically above the conveyor 20. In other examples, the x-ray receptor is aligned along a plane (e.g., a horizontal plane, a plane that extends transverse to the belt) and further aligned with the x-ray generator (not depicted) that is positioned along the same plane.

The imaging control system 68 may include and store information regarding operation of the x-ray such as position of the x-ray sources, the position of the x-ray receptors, and/or the energy of the x-ray radiation emitted by the x-ray source. The imaging control system can include a processor that retrieves instructions and data from a memory to execute instructions, and the imaging control system 68 can receive a data or signals from the detection elements of the x-ray receptor, process the data to generate images, output the images to a display or to the control system 200 (e.g., for example the imaging control system 68 generates signals for displaying data on a screen), and/or output data to the image storage unit. In certain examples, the imaging control system 68 transmits the image and data from the processor to the control system 200, external systems, networks, and/or devices and can also receive data from external systems, networks, and/or devices. In certain examples, the detection elements of the x-ray receptor are arranged in a plane and collectively have a rectangular shape (e.g., the detection elements are arranged in a four by eight grid pattern). In other examples, the detection elements of the x-ray receptor are arranged in a line that extends under the conveyor. In this example, the imaging control system 68 is configured to combine multiple signals from the detection elements as the corn C is conveyed past the x-ray receptor to thereby form a complete image of the corn C.

The x-ray source generates x-rays or x-ray radiation a certain portion of which are absorbed by the corn C or passes through the corn C. The radiation passing through the corn C is detected by the detection elements of the receptor(s). The amount of radiation that passes through the corn C depends on the density and/or water content of the components of the corn C such as husk, kernels, and cob.

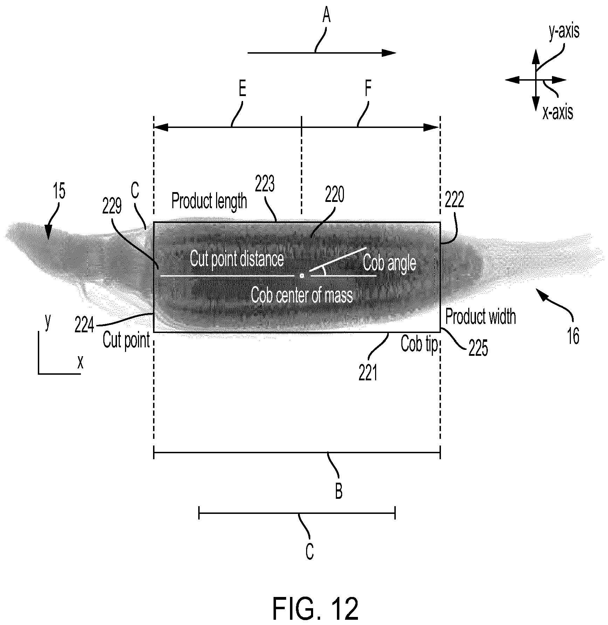

The variation in the intensity of x-ray radiation striking the x-ray receptor gives an indication of the features and structure of the corn C. In certain examples, the imaging system 67 presents the results in the form of an image that maps the intensity of x-rays falling on various parts of the receptor and thereby gives an indication of the distribution of density and/or water content attenuation values through the different components of the corn C that the x-ray beams have passed. An example two-dimensional image generated by the imaging system 67 of a corn C is depicted in Fig. 12 (described further herein below). In this example image, the husk has a lower density and water content than the kernels and cob and thus the pixels in the image for the husk are light in comparison to the darker pixels that correspond to the kernels and cob.

In certain examples, the x-ray can generate either two-dimensional (2D) or three-dimensional (3D) images, and in certain examples, x-ray can be multispectral. In certain examples, the x-ray generates a tomographic image of the corn C. The imaging control system 68 can be configured to process image(s), and different energy spectrums. In certain examples, the imaging system 65 captures silhouette images of the corn C. In some examples, the color of each pixel is the image generated by the x-ray of the imaging system 67 is based intensity of the x-rays received by the receptor. For instance, the husk has a lower density and thus a higher intensity of x-rays are received by a portion of the receptor such that the corresponding pixel in the image has a lighter color, and conversely, the cob has a higher density and thus a lower intensity of x-rays are received by a portion of the receptor such that the corresponding pixel in the image has a darker color. Note in certain examples, the imaging control system 68 can inverse the color of the pixels noted in the previous example. In certain examples, the imaging control system 68 is configured to identify and determine features of the corn C such as location of defects, color, and/or gaping, based on the images of the corn C.

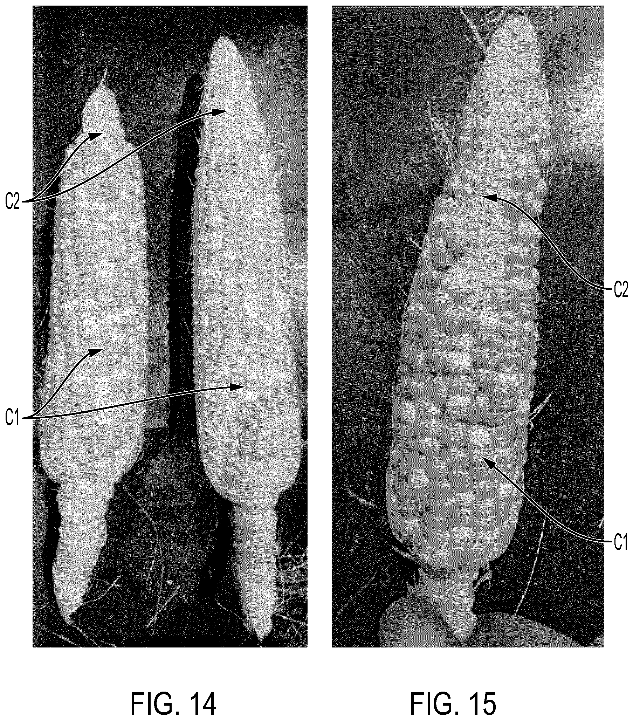

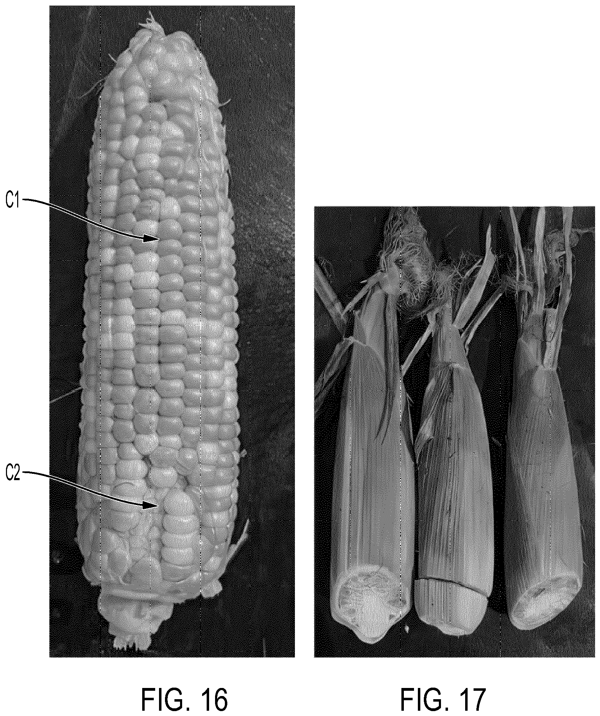

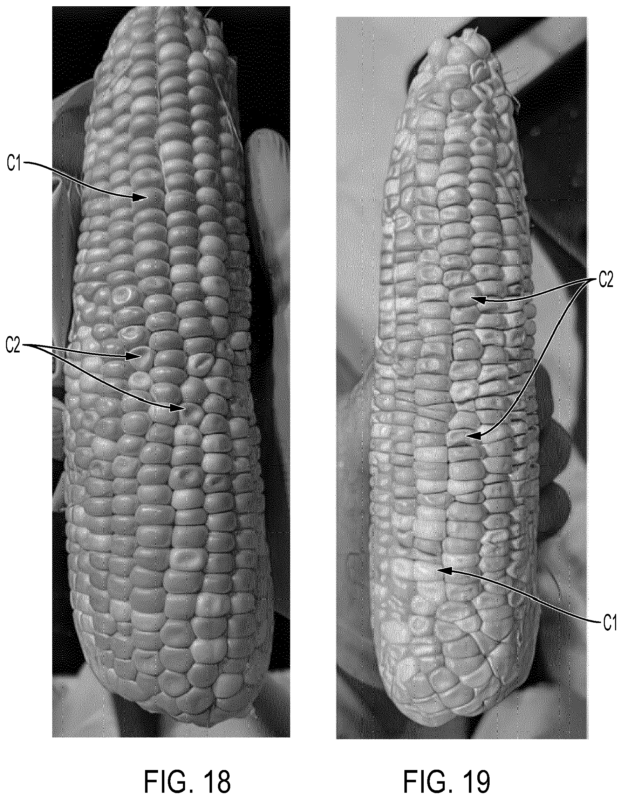

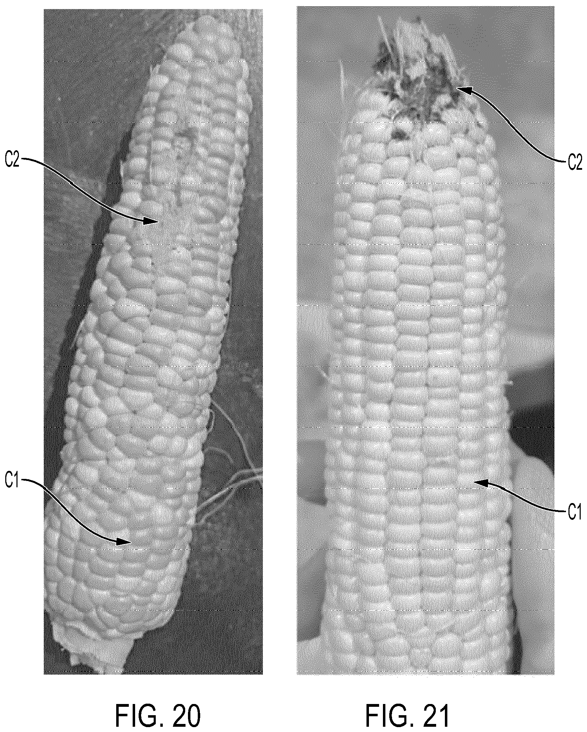

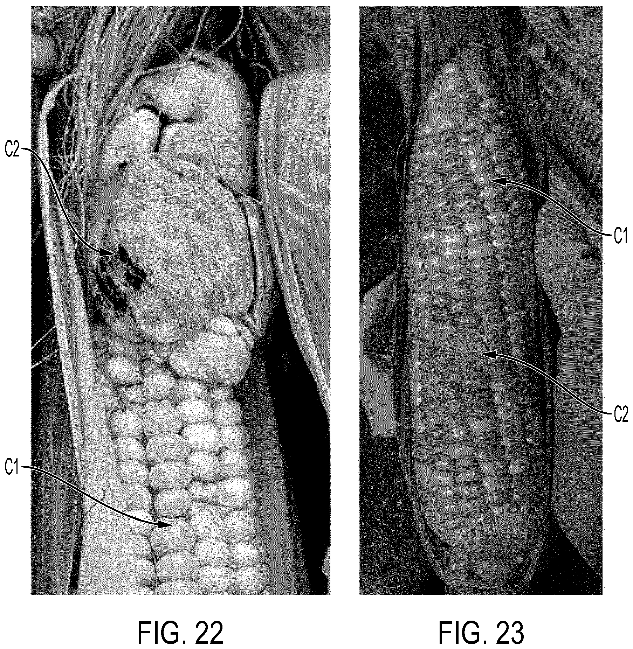

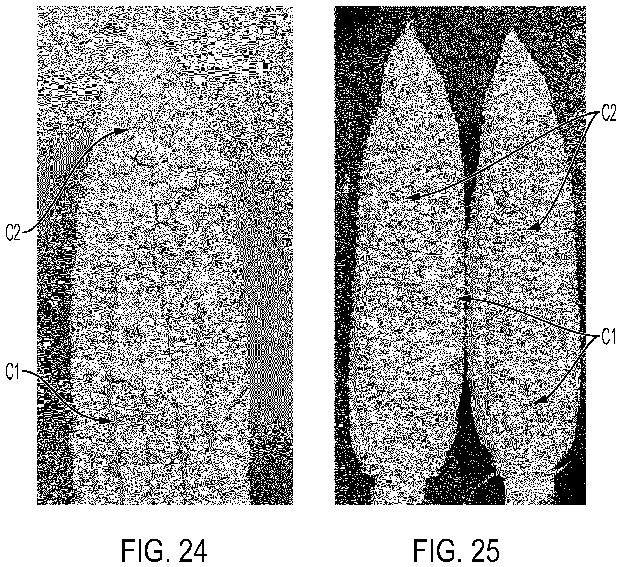

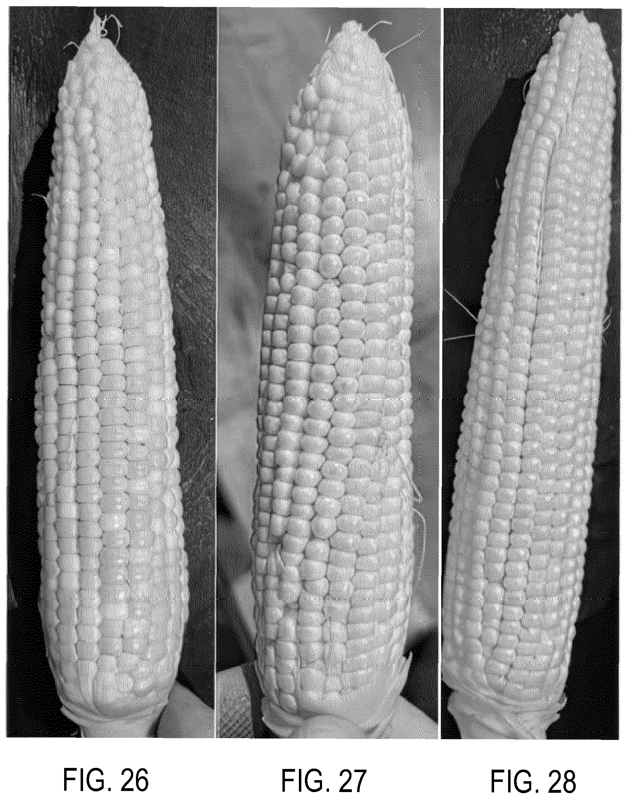

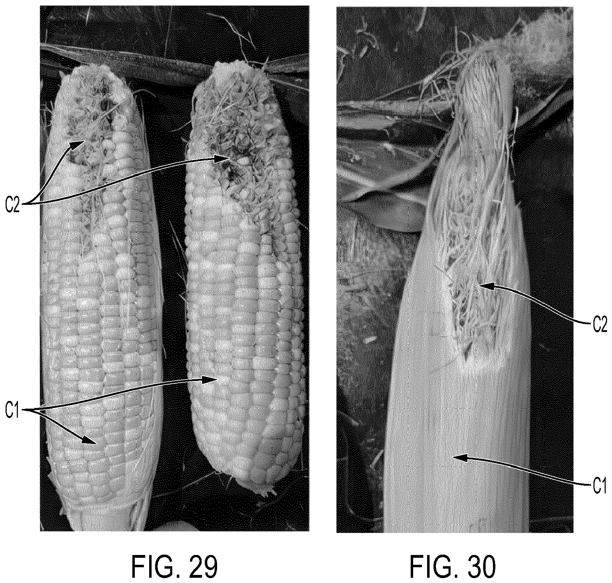

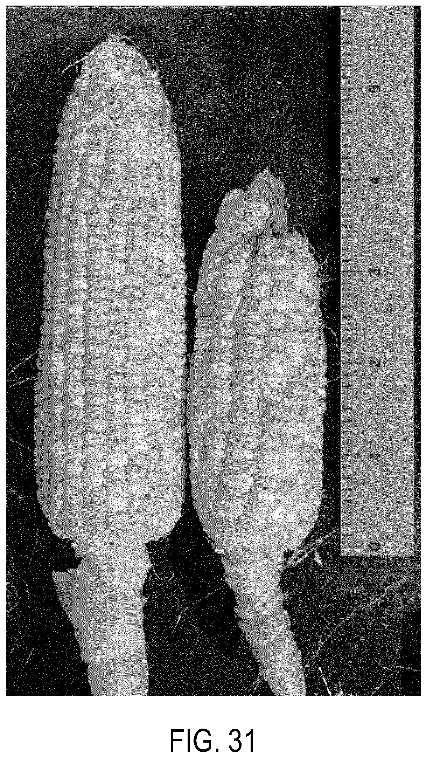

Figs. 14-31 are photos different ears of corn C having different defects that affect the quality of the corn. These defects can be identified by the system 10. Note that for reference and further clarity Appendix A filed herewith the present application includes black and white versions of Figs. 14-31. Fig. 14 depicts a corn C with normal corn kernels (see arrow C1) and a tip area of the cob devoid of kernels (see arrow C2). This defect is called tip blanking. Fig. 15 depicts a corn C with normal corn kernels (see arrow C1) and a body area of the cob devoid of kernels (see arrow C2). This defect is called body blanking. Fig. 16 depicts a corn C with normal corn kernels (see arrow C1) and a butt area of the cod devoid of kernels (see arrow C2). This defect is called butt blanking. Fig. 17 depicts corn C that have been mechanically damaged (see arrow C2) with large cuts or missing section due to the harvesting process. Figs. 18-19 depict corn C with normal corn kernels (see arrow C1) and kernels that are dented or dimpled due to improper growth (see arrow C2). These defects are called denting. Figs. 20-21 depict corn C with normal corn kernels (see arrow C1) and kernels that have been damaged by insects (see arrow C2). Fig. 22 depicts a corn C with normal corn kernels (see arrow C1) and smut at the tip of the cob (see arrow C2). Fig. 23 depicts a corn C with normal corn kernels (see arrow C1) and decayed kernels in the body section of the corn C (see arrow C2). Fig. 24 depicts a corn C with normal corn kernels (see arrow C1) and kernels that are dehydrated near the tip of the cob (see arrow C2). Fig. 25 depicts a corn C with normal corn kernels (see arrow C1) and kernels that are dehydrated along a section of the body (see arrow C2). Figs. 26-28 depicts corn C having immature, not fully developed kernels. Figs. 29-30 depict corn C with normal corn kernels (see arrow C1) and kernels that have been damaged by birds (see arrow C2). Fig. 31 depicts corn C having short and inadequate length.

In certain examples, the imaging control system 68 is configured to process the image to determine and identify edges, thicknesses, and/or defects of the corn C based on processing the pixels in the generated images. The imaging control system 68 may use any known processing modules such as edged detection modules, image reduction modules, de-noising modules, and the like for determining edges, thicknesses, outer limits of the good quality portions of the corn C, defects, color, gaping and/or the like. In certain examples, the x-ray imaging system is configured to determine the mass density of the corn C, a geometric centroid of the corn C area, and/or the center of gravity of the corn C. In other examples, multiple x-ray images may be taken of each corn C as the corn C is conveyed past the imaging zone of the x-ray at differing angles and the multiple resulting x-ray images are matched using a coordinate system. Reference is made to U.S. Patent Application Publication No. 2012/0307013, which is incorporated by reference in its entirety, for example mapping procedures, cutting tools, processing steps (e.g., determining the center of gravity), components, and/or features that can be combined with the example systems 10 of the present disclosure.

In certain examples, the imaging control system is configured to match the x-ray image of each corn C to determine the location of the corn C relative to the conveyor 20. This can be achieved, for instance, by determining the center of gravity of the corn C based on the x-ray image and the corresponding location of the center of gravity of the corn C on the conveyor 20 (e.g. imaging position).

Referring to Fig. 13, in certain examples, the image of the corn C (see example image of corn C depicted in Fig. 12) generated by the x-ray is used to locate the imaging position of the corn C on the conveyor 20. Fig. 13 depicts the conveyor 20 with opposing lateral sides 22 of the belt 21 and the longitudinal belt axis B2. The imaging control system 68 determines the center of gravity of the corn C (note in other examples the imaging control system 68 may determine another feature of the corn C such as the edge of kernels or beginning of shank to determine the imaging position of the corn C), and then the imaging control system 68 compares the location of the center of gravity in the image to known position data related to the conveyor and the imaging zone 69 of the x-ray relative to the conveyor 20. In one example, the x-ray generator and the x-ray receptor are fixed relative to each other and the conveyor 20, and image generated will be of the same imaging zone 69 on the conveyor 20 and have the same extents. In Fig. 13, the imaging zone 69 that corresponds with the extents of the generated image is depicted overlaid on the conveyor 20. The imaging zone 69 has a first axis 71 (e.g., x-axis), a second axis 72 (e.g., y-axis), and the point of intersection 70 of the axes 71, 72. Note that the example depicted the first axis 71 aligns with the longitudinal belt axis B2; however, in other example, the first axis 71 may be offset from the longitudinal belt axis B2. Prior to imaging the corn C and during initial calibration of the system 10, the imaging control system 68 is configured to determine the location of the axes 71, 72 and/or the point of intersection 70 relative to the belt 21 and the side 22 of the belt 21. In one example, the imaging control system 68 can determine that the first axis 71 of the imaging zone 69 aligned with the longitudinal belt axis B2, the second axis 72 is at an initial y-axis position relative to the belt 21 and the point of intersection 70. For purposes of example, the point of intersection 70 corresponds the imaging position (e.g., the center of gravity of the corn C) at initial an x-axis value 0.0 along the longitudinal belt axis B2 and at initial a y-axis value 0.0 along initial y-axis value 0.0 along a laterally extending y-axis 75 on the conveyor 20 (note the y-axis 75 in Fig. 13 aligns with the second axis 72). The imaging control system 68 further determines that location of the corn C relative to the conveyor 20 based on the generated image. For instance, the imaging control system 68 determines that the intersection 70 is at a coordinate datum (0,0) in the generated image and the center of gravity of the corn C is offset from the coordinate datum by one unit along each of the axes to a belt position coordinate of (1,1). Note in other examples the example coordinate (1,1) may correspond to the geometric centroid of the corn C. Thus, the imaging control system determines the imaging position of the corn C is offset by one unit along each of the axes 71, 72 of the conveyor 20 (see point 76) based on the offset in the image. In other examples, the location of the determined imaging position of the corn C corresponds to another feature or characteristic of the corn C determined by the imaging control system 68 such as a first row of kernels on the shank side of the cob (exemplarily denoted as location 229 on Fig. 12). Note that in other examples, such as when the detection elements of the x-ray receptor are arranged in a line, the imaging zone is generally a line and the control system 68 determines the position of the corn C on the belt 21 based on the linear imaging zone and position data from the conveyor 20.

In certain examples, the imaging control system 68 and/or the control system 200 is configured to configured to determine and track the movement of the corn C as the conveyor 20 moves the corn C in the downstream direction (arrow A). The control system 200 receives data from one or more encoders 23 (Fig. 8) of the conveyor 20 that correspond to movement of the belt 21 over a time period. The control system 200 then determines the current corn position of the corn on the conveyor 20 based on the imaging position of the corn C (as described above), the time period, and/or the position data from the encoder. For example, the images control system 68 determines that the imaging position is at coordinate (1,1) of the conveyor 20 which is communicated to the control system 200 and the encoder 23 sends data to the control system 200 regarding the belt 21 moved 15 units in a 5.0 second time period. In this example, the encoder would be used to track the coordinate frame and corn C location which respect to the coordinate frame. An encoder value at the instant the image was registered is recorded and the counts down time location to a downstream location. As such, the control system 200 determines that the current corn position of the corn is at coordinate (16,1). This current corn position is utilized by the sorting section 90 and/or the robot 91. Note that the control system 200 may continuously receive data from the encoder 23 and update the current corn position to thereby track the position of the corn C as it is conveyed by the conveyor 20. In certain examples, a light curtain or laser system (not depicted) is used with (or as a substitute for) the encoder 23 to generate position data. In one of these examples, as the leading end of the corn C enters a light plane generated by the light curtain system that is at a fixed and known location in the imaging system 67, the light curtain system generates and sends signals to the control system 68. Once the opposite trailing end of the corn C passes the light curtain system ceases generating the signals and the control system 68 processes the data to determine the location of the corn C including the ends. The control system 68 also utilizes other data (e.g., speed of the conveyor 20, time of conveyance), such that the real time position of the corn C can be determined.

In addition, the imaging control system 68 can also be configured to identify defects (see example corn C defects noted above and in Figs. 14-31) based on the processing the images generated by the imaging system 67. The imaging control system 68 may process the image using several different algorithms, image processing tools, and/or software modules note above. Furthermore the imaging control system 68 can be configured to determine a quality grade for the corn C (e.g., good quality corn with no defects, poor quality corn with one or more defects) without removing the husk. That is, the imaging control system 68 is configured to determine and identify defects in the cob and/or the kernels of the corn C without needing the operator to remove the husk. As such, the imaging system 67 is able to display the "hidden" or internal features or components of the corn C in the generated image. The present inventors have recognized that it is advantageous to determine the quality of the corn C and/or if defects in the corn C so that the corn can be properly processed by the system 10. For example, the operator may wish to inspect, discard, and/or allocate for other purposes corn C with poor quality. The operator may also wish to use and/or package good quality corn C for sale to consumers. As such, the present inventor developed the systems 10 described herein for determining the quality of the corn C and further processing the corn C accordingly to their determined quality. Defects in corn C can include tip-blanking, body blanking, butt blanking, denting, worm damage, smut, decay, dehydration, immaturity, bird damage, short ears, and the like. See example Figs. 14-31 for example defects in corn C.

To determine the quality grade of the corn C, the image of the corn C generated by the imaging system 67 is further processed by the imaging control system 68. As is briefly described above, the imaging system 67 generates an image of each corn C as the corn C pass through a the x-ray such image generated is a black and white image based on the intensity of the x-ray beam passing through the corn C. An example image generated by the x-ray is depicted in Fig. 12 (this Figure is described in greater detail hereinbelow). The lesser the intensity of the x-ray transmission through the corn C, the greater density of the respective region of the corn C. As such, the x-ray image of the corn C results in a mapping of the intensity detected over the area of the corn C by the control system 200. In certain examples, the detected intensity corresponds to the density of the corn C in a given location. For instance, for corn C (and other produce), regions of high moisture content generally have the highest density and thus appear dark on the image. In certain examples, when a fresh corn C is imaged by the x-ray, the kernels have the highest moisture content and therefore the highest density and thus appear dark on the image. The stalk, and core of the cob have a lesser density and thus appear light or are absent from the image. The husk material has a very low density and thus the husk does not appear in the image. The husk appears only minimally and is essentially ignored by the imaging control system 68. This causes images generated by the x-ray to appear as if the husk were removed (e.g., the x-ray image depicts an image of the kernels and cob without the husk shown in the image). In the event the kernels are damaged, decayed, dried, and/or missing the images generated by the x-ray will include areas of lower intensity and thus appear light on the image. Furthermore, the defective or damaged kernels will be visible in the image as irregularities compared to non-deformed kernels.

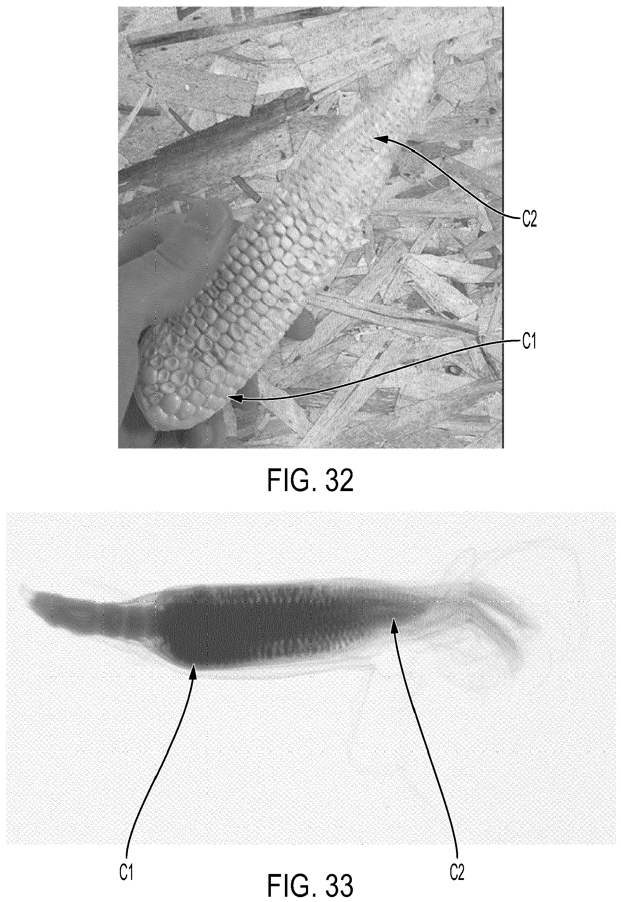

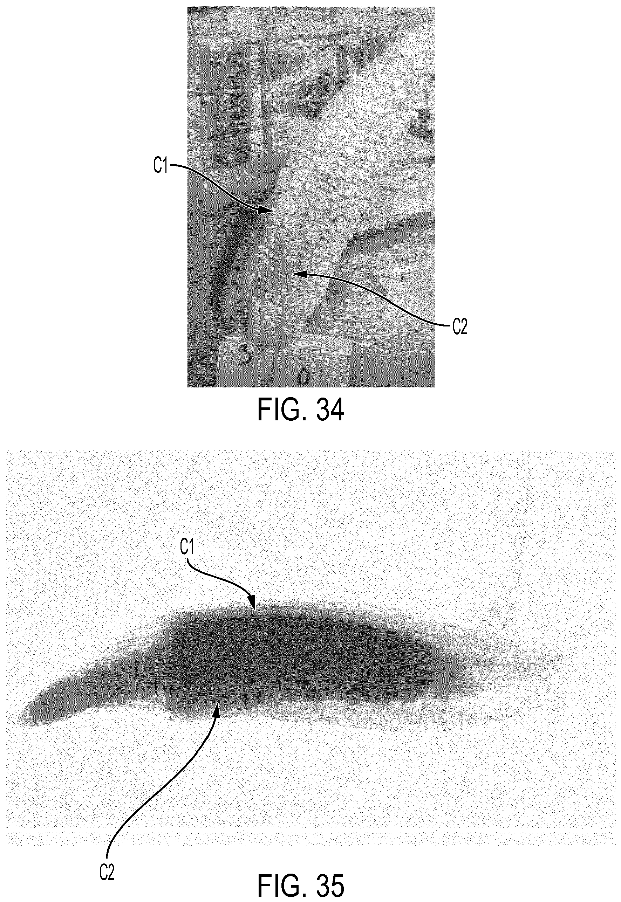

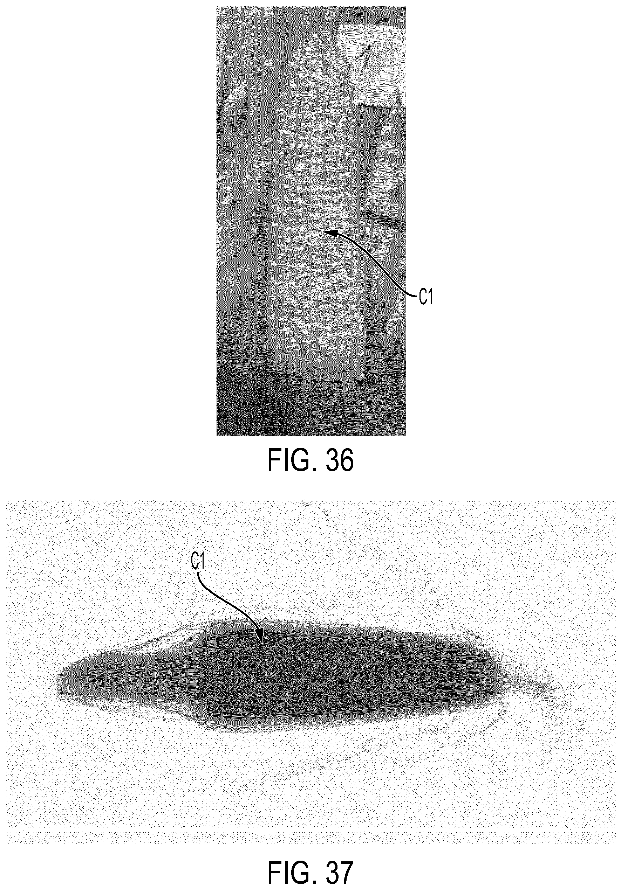

Figs. 32-37 depict example of ears of corn C with the corresponding x-ray image generated by the imaging system 67. Fig. 32 depicts a corn C with normal kernels (see arrow C1) and an area of the corn C with dehydrated kernels and tip blanking (see arrow C2). As such, as depicted in Fig. 33, the image of the corn C includes darker pixels in regions corresponding to the normal kernels (see arrow C1) and lighter pixels in regions corresponding to the portions of the corn C with defects (see arrow C2). Note that the husk and shank are also depicted with lighter pixels. Fig. 34 depicts a corn C with normal kernels (see arrow C1) and an area of the corn C with body dehydration and body blanking (see arrow C2). As such, as depicted in Fig. 35, the image of the corn C includes darker pixels in regions corresponding to the normal kernels (see arrow C1) and lighter pixels in regions corresponding to the portions of the corn C with defects (see arrow C2). Fig. 36 depicts a corn C with normal kernels (see arrow C1) and devoid of defects. As such, as depicted in Fig. 37, the image of the corn C includes a large section of darker pixels corresponding to the normal kernels (see arrow C1).

In one non-limiting example, the imaging control system 68 determines the quality grade of the corn C by determining a number of areas of suspected defects based on the generated images and determining a letter grade for the corn based on a look-up table stored in the memory. The defects are determined by the imaging control system 68 by processing the images. For example, the imaging control system 68 may compare the pixels within the image or an average pixel intensity of an area of pixels in the image to a predetermined pixel intensity threshold value set by the operator. If the imaging control system 68 determines that actual pixel intensity value is below the predetermined pixel intensity threshold, the imaging control system 68 determines that a defect is present. The imaging control system 68 sums the number of defects in the image and applies a quality grade to the to the corn. For example, the quality grade may include a letter grade such as "A" for 0-1 defects, "B" for 2-3 defects, "C" for 4-5 defects, or "D" for 6-7 defects that correspond to good quality or poor quality corn C, respectively. For example, a "C" quality grade may be associated with a corn C in the event that four defects are present in the corn C. As such, the control system 200 may mark this corn for discard from the system 10. In another example, a "B" quality grade may be associated with a corn C in the event that two defects is present in the corn C. As such, the control system 200 may mark this corn for routing to an alternative sorting section different than the sorting section 90. In another example, an "A" grade may be associated with a corn C with zero defects. As such, the control system 200 may mark this corn C for sorting in the sorting section 90.

The imaging control system 68 can further determine a desired or acceptable quality product region 221 (see Fig. 12) that is of good quality and devoid of defects. This determined product region 221 corresponds to a section/portion of the corn C that can be utilized the operator, for example as a salable portion, that is free of defects and/or irregularities. For example, the product region 221 corresponds to a portion of the corn C that has normal kernels (for instance Figs. 36-37). Based on the determined product region 221 the processing section 120 removes (e.g., cuts away) the other portions of the corn C (e.g., the shank, defective regions of the corn C). For instance, the operator may wish to cut the corn C such that the product region 221 is separated from the remainder of the corn C such as the end, defective areas, and/or the husk. The product region 221 is determined based on the location the defective areas of the image (as described above). In certain examples, the product region 221 corresponds to a region of the cob where the pixel intensity or density is above the predetermined pixel intensity threshold. In certain examples, the product region 221 is used to determine the center of mass of the corn C. In certain examples, the product region 221 is tracked by the control system 200 as the corn C is conveyed by the conveyor 20 after imaging. Furthermore, in certain examples, the imaging control system 68 may utilize programs, software modules, and/or imaging processing modules to determine features or data of the product region 221 such as the product width 222, the product length 223, the cutlines 224, 225, and/or the cut distances 226, 227. In certain examples, the predetermined pixel intensity threshold value for determining the product region 221 can be stored on the internal control system of the imaging control system 68 or the control system 200. The operator may also enter the product region 221 and/or the pixel intensity into the control system 200 and/or the memory system 204 via an input device 208 such as a touchscreen user input device.

In certain examples, to ensure the accuracy of product region 221 and the corresponding cutlines 224, 225, the processing system 202 is configured to determine a location of the transition between the good quality kernels and the shank of the corn C in the processed image of the corn C. This transition occurs at the location where there is abrupt narrowing and reduction of density in the corn C. The control system 200 includes an image processing algorithm that recognizes the location of the transition point and uses it to define a tail end point of the product region 221 and a tip end point of the product region 221. The image processing algorithm further processes these points to define a rectangle around the product region 221. As such, other sections of the system 10, such as the sorting section 90, can use the points or the rectangle for further processing such as vertically picking the corn C off the conveyor 20 or cutting the corn C (described further herein).

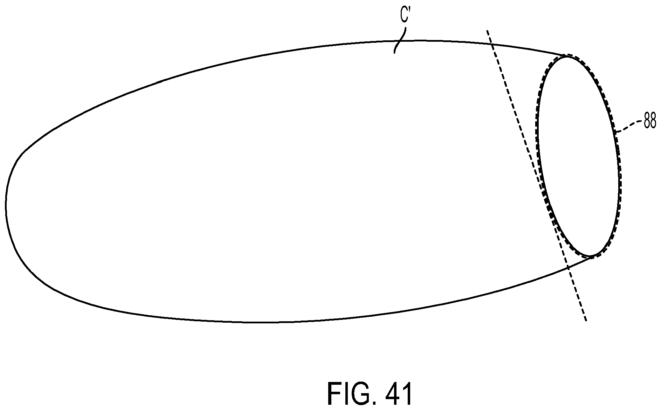

In one non-limiting example as depicted in Fig. 12, the product region 221 includes a product width 222 and a product length 223. The product length 223 is bound between a first cutline 224 and an opposite second cutline 225, and in the example depicted in Fig. 12 the product region 221 has length B between the cutlines 224, 225. Note that in certain examples the second cutline 225 is not determined by the control system 200. In one example, the cutlines 224, 225 are determined by the imaging control system 68 along a section of the image along a y-axis that is free of defects and is nearest to the tip end 16 of the corn C. The image control system 68 can also determine a corresponding cutline plane (see dashed line 88 on Fig. 41 depicting the cutline plane intersecting the cut ends of a processed corn C') for the corn C relative to another determined position of the corn C (e.g., the center of gravity as described above such that the cutline plane can be utilized by the sorting section 90 and/or the processing section 120. In one non-limiting the instance the center of gravity is at a coordinate datum (0,0) in the generated image and the cutline 225 is at a cut coordinate (0, 5) and as such the imaging control system 68 and/or the control system 200 controls the sorting section 90 and/or processing section 90 such that the corn C is cut along the cutline plane (described further herein).

In certain examples, the first cutline 224 is predetermined and set by fixing the location of a cutting blade 128 (described below). In a non-limiting example, the product region 221 may have a length C between the cutlines 224, 225 that is less than length B such that the defect or defective area is excluded from the product region 221. The processed image data can also include a first cut distance 226 from the center of mass 220 to the first cutline 224 and a second cut distance 227 from the center of mass 220 to the second cutline 225. In other examples, the processing system 202 includes an algorithm of the control system 200 that determines the cut distances 226, 227.

In another non-limiting example, the imaging control system 68 and/or the control system 200 can accept or reject a corn C based on comparing the image to threshold data stored on the memory system 204. Note that the threshold data may be entered into the control system 200 by the operator. For instance, size threshold data can include minimum length value, maximum length value, minimum width value, and/or maximum width value related to the desired dimensions of the product region 221. For example, if the product length 223 is less than the minimum length value the control system 200 marks the corn C for discard or assigns a poor quality grade (e.g., "D" grade).

In certain examples, the imaging section 60 and/or the imaging device 65 includes an imaging control system (not depicted; described above) configured to process each image generated by the imaging device 65. In these examples, the In this example, the in imaging control system analyzes each image using a built-in image processing software and transmits the images, processed image, data sets, position data, and/or processed image data to the control system 200 (see Fig. 11) for further processing and utilization other sections of the system 10. The control system 200 that processes the image generated by the imaging section and/or the further processes the imaged processed by the imaging control system 68 noted below is described further herein below.

Referring now to Fig. 11, a schematic diagram of an example control system is depicted in greater detail. Certain aspects of the present disclosure are described or depicted as functional and/or logical block components or processing steps, which may be performed by any number of hardware, software, and/or firmware components configured to perform the specified functions. For example, certain embodiments employ integrated circuit components, such as memory elements, digital signal processing elements, logic elements, look-up tables, or the like, configured to carry out a variety of functions under the control of one or more processors or other control devices. The connections between functional and logical block components are merely exemplary, which may be direct or indirect, and may follow alternate pathways.

In certain examples, the control system 200 communicates with each of the one or more components of the system 10 via a communication link 201, which can be any wired or wireless link. The control system 200 is capable of receiving information and/or controlling one or more operational characteristics of the system 10 and its various sub-systems by sending and receiving control signals via the communication links 201. In one example, the communication link 201 is a controller area network (CAN) bus; however, other types of links could be used such as TCP/IP, ethernet, and communications protocol. It will be recognized that the extent of connections and the communication links 201 may in fact be one or more shared connections, or links, among some or all of the components in the system 10. Moreover, the communication link 201 lines are meant only to demonstrate that the various control elements are capable of communicating with one another, and do not represent actual wiring connections between the various elements, nor do they represent the only paths of communication between the elements. Additionally, the system 10 may incorporate various types of communication devices and systems, and thus the illustrated communication links 201 may in fact represent various different types of wireless and/or wired data communication systems.

The control system 200 may be a computing system that includes a processing system 202, memory system 204, and input/output (I/O) system 203 for communicating with other devices, such as input devices 208 and output devices 207, either of which may also or alternatively be stored in a cloud 209. The processing system 202 loads and executes an executable program 205 from the memory system 204, accesses data 206 stored within the memory system 204, and directs the system 10 to operate as described in further detail below.

The processing system 202 may be implemented as a single microprocessor or other circuitry, or be distributed across multiple processing devices or sub-systems that cooperate to execute the executable program 205 from the memory system 204. Non-limiting examples of the processing system include general purpose central processing units, application specific processors, and logic devices.

The memory system 204 may comprise any storage media readable by the processing system 202 and capable of storing the executable program 205 and/or data 206. The memory system 204 may be implemented as a single storage device, or be distributed across multiple storage devices or sub-systems that cooperate to store computer readable instructions, data structures, program modules, or other data. The memory system 204 may include volatile and/or non-volatile systems, and may include removable and/or non-removable media implemented in any method or technology for storage of information. The storage media may include non-transitory and/or transitory storage media, including random access memory, read only memory, magnetic discs, optical discs, flash memory, virtual memory, and non-virtual memory, magnetic storage devices, or any other medium which can be used to store information and be accessed by an instruction execution system, for example.

Note that the example control system 200 features described herein below are described with reference to the image, processed image, and/or other data received from the imaging section 60 and/or the imaging control system 68 thereof. However, a person of ordinary skill in the art will recognize that in other examples, the control system 200 can include algorithms, programs, software modules, look-up tables, and/or the like in the memory system 204 such that the image can be processed by the control system 200. The imaging system 60 is in communication with the control system 200 such that the control system 200 receives images, data, and/or signals corresponding the imaged corn from the imaging system 67.

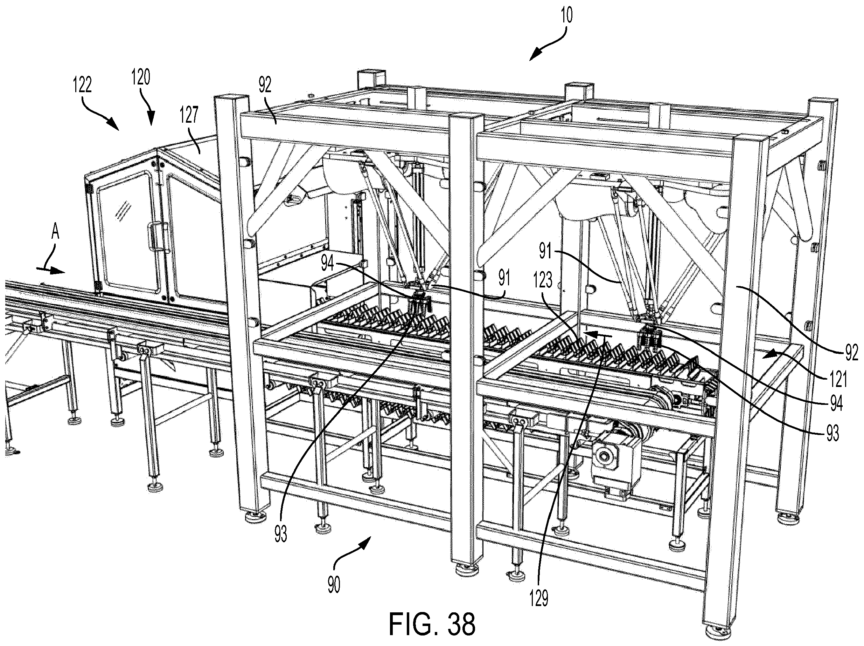

Referring now to Figs. 38-40, an example sorting section 90 is depicted in more detail. The sorting section 90 receives the corn C from the conveyor 20 of the imaging section 60 and the conveyor 20 extends into the sorting section 90. In another examples, the robot or other mechanical handing system could place the corn C onto one or more conveyors. The sorting section 90 is in communication with the control system 200 and/or the imaging control system 68 and is for moving and sorting the corn C based on the determinations made by the control system 200 such as the quality grade of the corn C and/or the data noted above. The sorting section 90 includes one or more sorting devices that are configured to move the corn C off the conveyor 20 and/or sort the corn C. In the example depicted in Fig. 38, the sorting section 90 includes two sorting devices which are independently operable robot 91 having a moveable arm. Each robot 91 is coupled to a frame 92 such that the robots 91 are above the conveyor 20 and therefore can engage the corn C as the conveyor 20 conveys the corn C into the sorting section 90. The robots 91 are configured to move along three axes via one or more actuators and move in any direction. Each robot 91 includes a gripper, e.g. suction cup or gripping fingers 93, for handling the corn C. The sorting device can be any suitable device for sorting the corn C. In one alternative embodiment, the sorting device is a series of actuated paddles that actuate to thereby direct corn C along one or more secondary conveyors. In this example, the secondary conveyors convey the corn C to different processing sections that process the corn C or to a discard area where the corn C is discarded. In other examples, the sorting device could include smart conveyors and diverters. In certain examples, the robot 91 has on more actuators, such as air cylinder, motor, linear motor, traditional motor, or solenoid, for moving components of the robot 91.

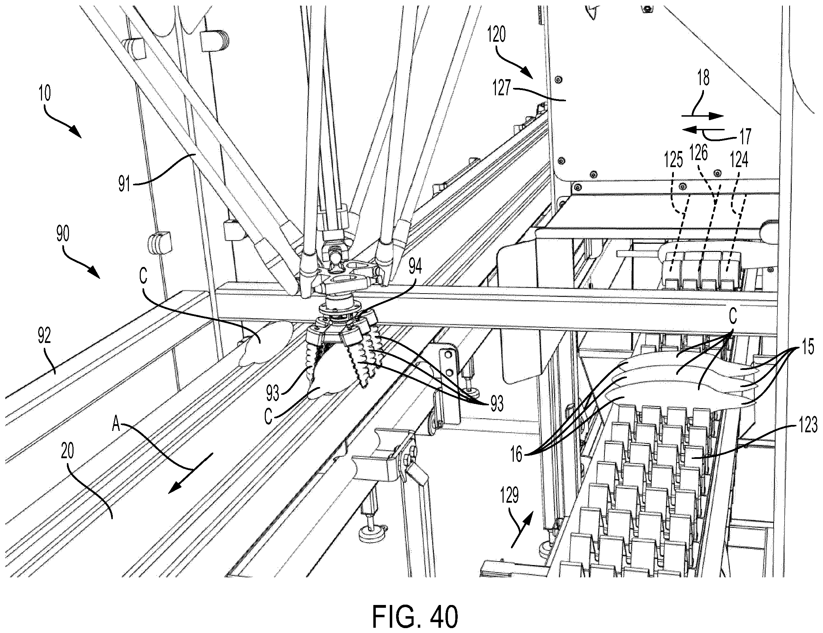

Figs. 39-40 depicts an example operational sequence of the robot arm 91 in more detail. Fig. 39 depicts the conveyor 20 conveying the two corns C in the downstream direction (arrow A) into the sorting section 90. The corn C are in two different rows, and the first robot arm 91 is vertically above the conveyor 20 and is in a rest position. Note that the corn C are schematically depicted with the upper half of the corn C removed to expose the center cross-sectional area of the corn C. The sorting section 90 and the first robot arm 91 are in communication with the control system 200 and thus, the first robot arm 91 can be controlled by the control system 200 based on the images and/or data noted above as the corn C are conveyed in to a zone in which the first robot arm 91 can move and/or engage with corn C. For example, the control system 200 utilizes the data from the imaging control system 68 and/or the control system 200, such as the image of the corn and/or data related to the corn C (e.g., characteristic of the corn C determined by the imaging control system such as imaging position, current corn position, defects, quality grade, product region, and/or cutline plane, which are described above), to thereby move the first robot arm 91 and actuate the fingers 93 to precisely grasp the corn C (see Fig. 15). The control system 200 the controls the first robot arm 91 to thereby place the corn C into the processing section 120 for processing (see Fig. 40 and described further herein).

In certain examples, the control system 200 controls the first robot arm 91 to thereby rotate the corn C such that the ends of the corn C are orientated in the same direction. Fig. 40 depicts the first robot arm 91 rotating the corn C such that the tip ends 16 are orientated in a first direction 17 and the tail ends 16 are oriented in an opposite second direction 18 on a conveyor 123 of the processing system 130 that extends along the sorting section 90 and the conveyor 20.

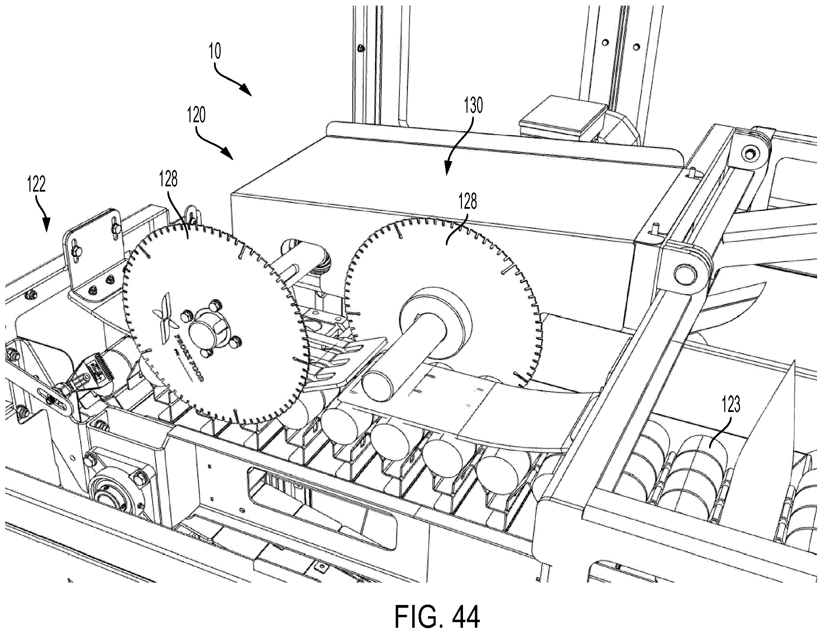

Furthermore, in certain examples, the control system 200 controls the first robot arm 91 to thereby place the corn C on the conveyor 123 of the processing section 120 such that one or more cutline planes or cutlines 224, 225 of the product region 221 of the corn C (see Fig. 12) aligns with one or more blade cut plane(s) 124, 125 of the processing section 120. The blade cut planes 124, 125 are partially depicted in Fig. 15 as evenly dashed lines. In this example, the processing section 120 cuts the corn C along the determined cutline plane (described above) by placing the corn C onto the processing section 120 such that the determined cut plane aligns with the blade cut plane 124 of a first blade 128 (Fig. 44). As such, the corn C exits the processing section with the tip end 16 cut from the corn C (see Fig. 41). In certain examples a second blade (Fig. 44) cuts the corn C along the second blade cut plane 125 such that corn C is a processed food product / corn C' (the processed corn C' shape is schematically depicted in Fig. 41).

In certain examples, the first robot arm 91 is capable of placing the corn C in precision locations on the conveyor 123 of the processing section 120 based on the image of the corn C and/or the data determined by the control system 200 (e.g., current corn position, cutline planes, cutlines 224, 225), and calibration data of the first robot arm 91. The calibration data can include arm position data (e.g., x-axis values, y-axis values) can include location data of a center of a grabber 94 of the first robot arm 91 relative to the conveyor 20 while in the rest position. As such, the control system 200 can move the first robot arm 91 such that the center point of the grabber 94 aligns with the center of mass 220 of the corn C when the corn C is in a current corn position (as described above). The fingers 93 then engage and vertically lift the corn C off the conveyor 20. The control system 200 can then move the first robot arm 91 such that the center point of the grabber 94 is offset from a center plane 126 (partially depicted as dash-dot line on Fig. 15) such that the one or more cutlines 224, 225 or cutline planes for the corn C align with the blade cut plane(s) 124, 125.

In certain examples, the control system 200 can utilize the image and the data determined by the imaging control system 68 and/or the control system 200 to determine X,Y,Z coordinates values of each corn C as the corn C is conveyed through the system 10. The robot 91 uses the coordinates to pick the corn C at the proper location, and place the cob C onto the cob saw at a corresponding proper X,Y,Z coordinates such that the corn C is properly cut as described herein. Then the robot 91 returns to a home position, or picks the next corn C, if available. In certain examples, the robot 91 includes a control system that calculates corresponding robot axis positions such that the gripper will be located at any given X,Y,Z coordinates. In this example, the robot control system translates the desired position, velocity, acceleration and jerk values of the gripper tool into the respective values needed for each robot axis. The Z coordinate may be related to the location of the corn C on or above the conveyor 20 and/or conveyor 123 as the corn C is moved and processed as described above.

Referring back to Fig. 38, the processing section 120 is depicted extending longitudinally along the sorting section 90. The processing section 120 includes a processing system 130 configured to process the corn C and thereby dispense a processed corn that corresponds to the product region 221 (described above). The processing system 130 includes a first end 121 and an opposite second end 122. The conveyor 123 extends between the ends 121, 122 and is configured to convey the corn C in first processing direction 129 through the processing section 120. Note that in this example the first processing direction 129 is opposite the downstream direction (see arrow A). The processing section 120 need not be positioned as depicted in Fig. 38, and instead, other example processing sections 120 may extend away from the sorting section and/or the conveyor 123 may be orientated differently than depicted. The processing section 120 has a processing system 130 configured to process the corn C, and for the example system 10, the processing section 120 is configured to cut the corn C along the cutline plane(s) as corresponding to the cutlines 224, 225 determined by the control system 200 (as described above). As such, the corn C is dispensed from the processing section 120 in the desired shape and portion for further processing by other system or workers. In one specific non-limiting example, the corn C is dispensed from the processing section 120 with the ends cut off such that the processed corn C has a generally truncated cylindrical shape (see Fig. 41 which depicts a schematic view of a processed corn C with the ends cut off). Husking is done after cutting on the cob saw, by a mechanical husking machine, then is packed onto trays manually or by robots. As such, in this example the desired salable portion of the corn C are free from defects and irregularities, has a desired quality, and/or are generally the same shape (see Fig. 41).

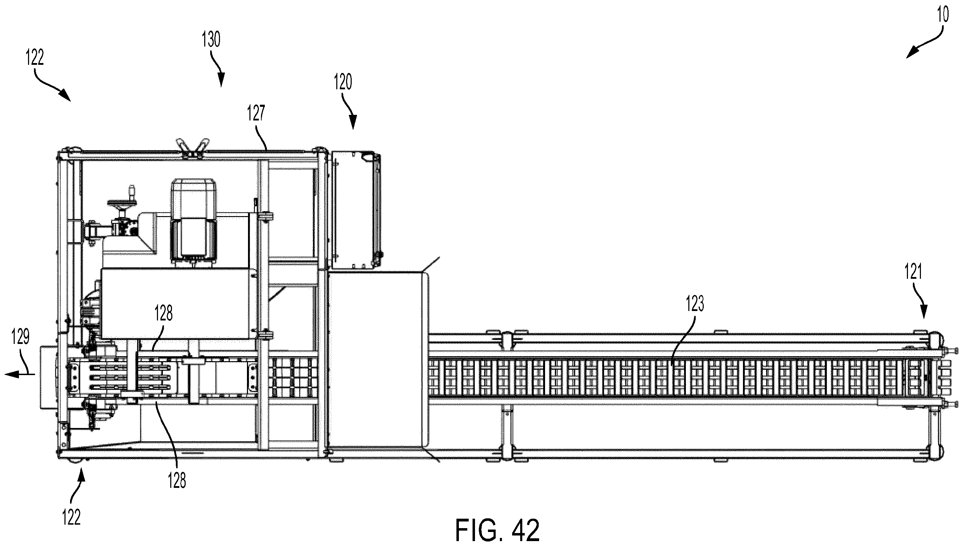

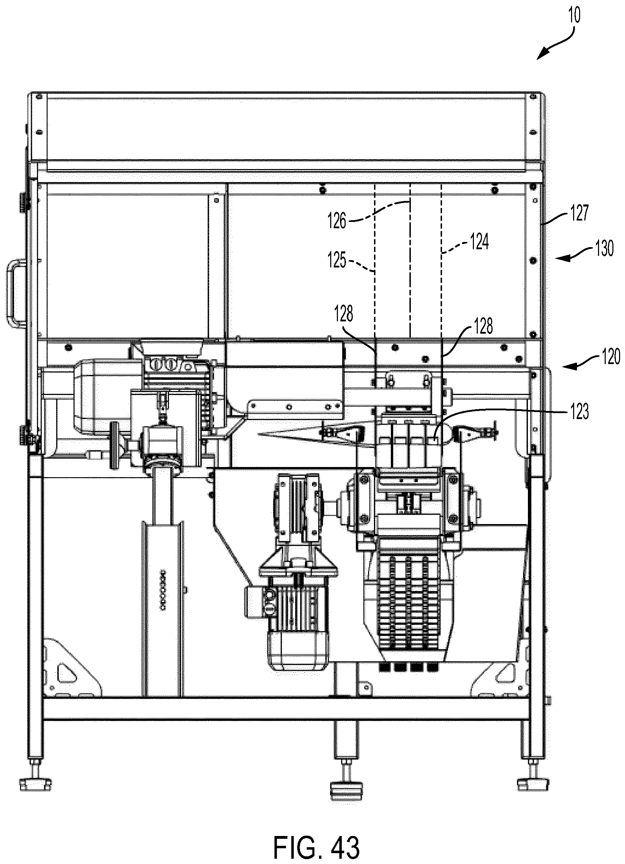

Figs. 42-44 depict the processing section 120 in more detail. The processing section 120 includes an enclosure 127 in which one or more cutting blades 128 configured to cut the corn C. The cutting blades 128 are positioned in operable association with the conveyor 123 such that the conveyor 123 is not cut by the blades 128 as the corn C is cut. The blades 128 are rotated by an actuator (e.g., motor) that is controlled by the control system 200. In one example, the control system 200 actuates the actuator to thereby rotate the cutting blades 128 at all times during operation of the system 10. In other examples, the control system 200 actuates the actuator to rotate the cutting blades 128 at time when the corn C is moved in close proximity and past the cutting blades 128. In this example, a sensor senses present and/or absence of the corn C near the cutting blades 128 or the control system 200 determines the position of the corn C on the conveyor 123 based on the data noted above and second encoder data from an encoder of the conveyor 123. In one example, one of the cutting blades 128 is movable relative to the another cutting blade 128 that is fixed in a cut position while the blades 128 are actuated.

The conveyor 123 can be any type of conveyor that is capable of transporting the corn C to the blades 128. In the example depicted in Figs. 42-44, the conveyor 123 includes an endless chain conveyor that defines grooves between adjacent chains in which the corn C are positioned and held as the corn C are conveyed in the first processing direction 129.

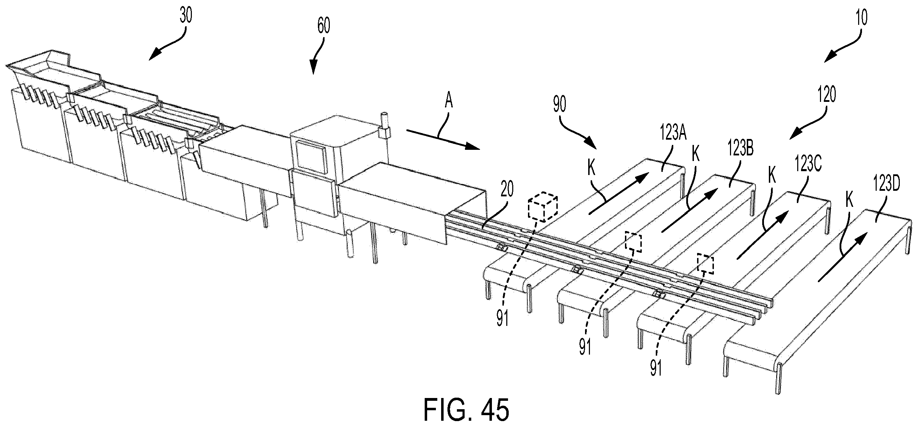

Referring now to Fig. 45, another example system 10 of the present disclosure is schematically depicted. The system 10 includes a conveyor 20 which the food products are conveyed in four rows in the downstream direction (arrow A). The sorting section 90 includes three sorting devices, such as robot arms 91 (depicted as dashed boxes), and each robot arm 91 is configured to move the corn food products from the conveyor 20 onto one of the conveyor 123A-D of the processing system 120. Each conveyor 123A-D conveys the corn C thereon to a different processing device (e.g., Kernel cutting, cobette cutting and packaging, loose corn packaging, tray packaging, waste.)As such, the control system 200 is configured to sort the corn C based on the quality of the corn C or other factors (e.g., large size corn C are moved onto the first conveyor 123A while small size corn are moved onto a second conveyor 123B) determined by the control system 200. Note the conveyor 20 automatically dispenses corn C remaining on the conveyor 20 to the conveyor 123D. The system will determine which conveyor the corn should be dispensed mechanically to for further processing based on customer specified requirements.

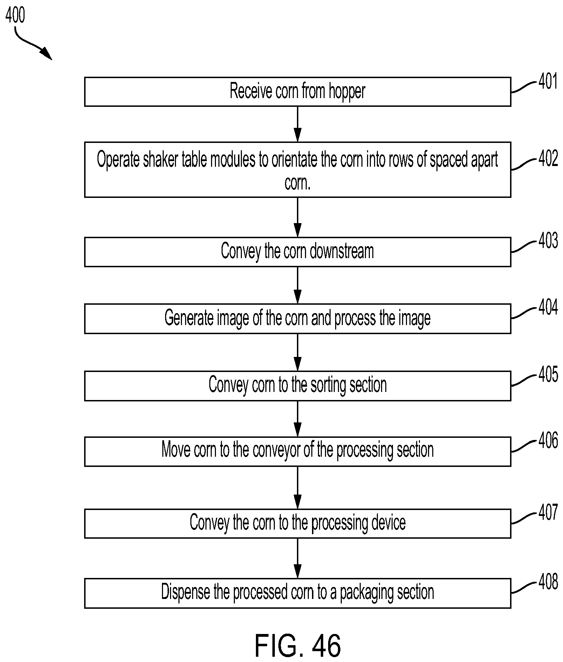

With reference to Fig. 46, an example process/method 400 for operating the system 10 is described hereinbelow. Note that the example process flow 400 can include other concepts and sequence steps and features of the example systems 10 noted above.

The example process flow 400 depicted in Fig. 46 is for processing a food product such as corn. The process flow 400 begins (at step 401) with the system 10 receiving a plurality of corn C into the input section 30 from a source. In certain examples, the source is a hopper that stores a large quantity of corn C therein and dispenses the corn C to the input section 30 at a desired flow rate or based on control signals received from the control system 200. Before and/or during receiving the corn, the shaker table modules 33 are operated (at step 402) such that the corn C is moved in the downstream direction (arrow A) and separated into rows of corn C that are oriented in a lengthwise orientation. At step 403 the corn C are dispensed from the input section 30 to the conveyor 20 of the imaging section 60 and the conveyor 20 conveys the corn C in the downstream direction (arrow A). The imaging section 60 generates an image of the corn C at step 404 and the image is processed by the internal control system of the imaging system 60 and/or the control system 200 to thereby determine characteristics (e.g., center of mass), quality grades, and/or features of the corn C. The internal control system of the imaging system 60 and/or the control system 200 is further configured to determine a desired product region of the corn C to be cut away from the remainder of the corn C.

At step 405, the conveyor 20 conveys the corn C to the sorting section 90 and the sorting section 90 engages and moves the corn C off the conveyor 20 to the conveyor 123 of the processing section 120 (at step 406). As part of moving the corn C to the conveyor 123, the robot arm 91 of the sorting section 90 may orientate the corn C is a desired direction and/or align one or more cutlines 224, 225 related to the determined product region 221 with one or more blade cut planes 124, 125. The conveyor 123 then conveys the corn C (at step 407) to the processing devices, e.g., cutting blades 128, that process, e.g., cut the corn C as indicated by the control system 200 and the determined product region 221. At step 408, the processed corn is dispensed to a packaging section (not depicted) where workers manually remove remaining husk and waste from the processed corn. The processed corn is then placed into containers and plastic wrap is applied.

In certain examples, a food processing system for processing a food product includes an imaging system for generating an image of the food product and a control system configured to process the image of the food product and determine a product region related to the food product. A robot is configured to move the food product into a processing system, and the processing system is configured to cut the food product and dispense a processed food product that corresponds to the product region.

Optionally, a conveyor is configured to convey the food product to the robot, and wherein the robot is configured to move the food product off the conveyor and further place the food product into the processing system. Optionally, the processing system includes a processing conveyor onto which the food products are received and one or more blades configured to cut the food product and thereby form the processed food product as the processing conveyor conveys the food product past the one or more blades. Optionally, the processing system is configured to determine a cutline plane for the food product, the one of blades defines a blade cut plane, and the robot is configured to move the food product into the processing system such that the cutline plane aligns with the blade cut plane. Optionally, the processing system includes two spaced apart blades that are configured to cut opposite ends from the food product. Optionally, during actuation of the blades one of the blades is movable relative to the other blade which remains fixed in a cut position.

In certain examples, a food processing system for processing a plurality of food products includes an imaging system having an x-ray for generating an image of each food product of the plurality of food product and a control system configured to process the image of each food product and determine a cutline for the image and a corresponding cutline plane for each food product along which the food product is cut. A conveyor is configured to convey each food product through the food processing system and a robot is configured to move each food product from the conveyor into a processing system. The processing system includes a processing conveyor onto which each food product is received from the robot. A blade is configured to cut each food product based on the respective cutline plane for each food product and the processing conveyor conveys the food product past the one or more blades such that each food product is a processed food product.

Optionally, the plurality of food products are produce. Optionally, the plurality of food products are ears of corn. Optionally, the robot is configured move each ear of corn onto the processing conveyor such that a tip end of each ear of corn is oriented in a first direction and a tail end of each ear of corn is oriented in a second direction opposite the first direction. Optionally, the processing includes a first blade configured to cut each ear of corn along the cutline plane determined for each ear of corn and a second blade configured to cut off a tail end of each ear of corn. Optionally, the first blade defines a blade cut plane and the robot is configured to move each ear of corn onto the processing conveyor such that the cutline plane for each ear aligns with the blade cut plane. Optionally, during actuation of the blades the first blade is movable relative to the second blade which remains fixed in a position relative to the first blade.

In certain examples, a food processing system for individually processing ears of corn includes an imaging system having an x-ray for generating an image of each ear of corn. A control system is configured to receive the image for each ear of corn from the imaging section and process the image to identify defects in the ear of corn and determine a product region of the ear of corn based on the identified defects. A conveyor is configured to convey each ear of corn through the food processing system. A robot is configured to move each ear of corn from the conveyor into a processing system. The processing system includes a processing conveyor onto which each ear of corn is received from the robot and a blade configured to cut each ear of corn based on the product region determined for each ear of corn such that each ear of corn is a processed ear of corn that corresponds to the product region.

Optionally, the control system is configured to determine an imaging position on the conveyor for each ear of corn based on the generated image for each ear of corn and further receive position data from the conveyor as the conveyor conveys the ears of corn in a downstream direction. The control system is configured to track position of each ear of corn along the conveyor based on the imaging position and the position data such that the robot can move each ear of corn from the conveyor to the processing conveyor. Optionally, the processing system includes a first blade configured to cut each ear of corn along a cutline plane determined for each ear of corn by the control system and a second blade configured to cut off a tail end of each ear of corn. Optionally, the first blade defines a blade cut plane and the robot is configured to move each ear of corn onto the processing conveyor such that the cutline plane for each ear aligns with the blade cut plane. Optionally, during actuation of the blades the first blade is movable relative to the second blade which remains fixed in a position relative to the first blade. Optionally, the robot is configured move each ear of corn onto the processing conveyor such that a tip end of each ear of corn is oriented in a first direction and a tail end of each ear of corn is oriented in a second direction opposite the first direction.

Citations to a number of references are made herein. The cited references are incorporated by reference herein in their entireties. In the event that there is an inconsistency between a definition of a term in the specification as compared to a definition of the term in a cited reference, the term should be interpreted based on the definition in the specification of the present disclsoure.

In the present description, certain terms have been used for brevity, clarity, and understanding. No unnecessary limitations are to be inferred therefrom beyond the requirement of the prior art because such terms are used for descriptive purposes and are intended to be broadly construed. The different apparatuses, systems, and method steps described herein may be used alone or in combination with other apparatuses, systems, and methods. It is to be expected that various equivalents, alternatives and modifications are possible within the scope of the appended claims.

The functional block diagrams, operational sequences, and flow diagrams provided in the Figures are representative of exemplary architectures, environments, and methodologies for performing novel aspects of the disclosure. While, for purposes of simplicity of explanation, the methodologies included herein may be in the form of a functional diagram, operational sequence, or flow diagram, and may be described as a series of acts, it is to be understood and appreciated that the methodologies are not limited by the order of acts, as some acts may, in accordance therewith, occur in a different order and/or concurrently with other acts from that shown and described herein. For example, those skilled in the art will understand and appreciate that a methodology can alternatively be represented as a series of interrelated states or events, such as in a state diagram. Moreover, not all acts illustrated in a methodology may be required for a novel implementation.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

The following clauses set out features of the invention which may not be presently claimed but which may form the basis for amendments or future divisional applications.

### CLAUSES

1. A food processing system for processing a food product, the food processing system comprising:
   an imaging system for generating an image of the food product;
   a control system configured to process the image of the food product and determine a product region related to the food product; and
   a robot configured to move the food product into a processing system, wherein the processing system is configured to cut the food product and dispense a processed food product that corresponds to the product region.
2. The food processing system according to clause 1, further comprising a conveyor configured to convey the food product to the robot, and wherein the robot is configured to move the food product off the conveyor and further place the food product into the processing system.
3. The food processing system according to clause 2, wherein the processing system includes:
   a processing conveyor onto which the food products are received; and
   one or more blades configured to cut the food product and thereby form the processed food product as the processing conveyor conveys the food product past the one or more blades.
4. The food processing system according to clause 3, wherein the processing system is configured to determine a cutline plane for the food product;
   wherein the one of blades defines a blade cut plane; and
   wherein the robot is configured to move the food product into the processing system such that the cutline plane aligns with the blade cut plane.
5. The food processing system according to clause 3, wherein the processing system includes two spaced apart blades that are configured to cut opposite ends from the food product.
6. The food processing system according to clause 5, wherein during actuation of the blades one of the blades is movable relative to the other blade which remains fixed in a cut position.
7. A food processing system for processing a plurality of food products, the food processing system comprising
   an imaging system having an x-ray for generating an image of each food product of the plurality of food products;
   a control system configured to process the image of each food product and determine a cutline for the image and a corresponding cutline plane for each food product along which the food product is cut;
   a conveyor configured to convey each food product through the food processing system; and
   a robot configured to move each food product from the conveyor into a processing system;
   wherein the processing system includes a processing conveyor onto which each food product is received from the robot and a blade configured to cut each food product based on the respective cutline plane for each food product, and wherein the processing conveyor conveys the food product past the one or more blades such that each food product is a processed food product.
8. The food processing system according to clause 7, wherein the plurality of food products are produce.
9. The food processing system according to clause 7, wherein the plurality of food products are ears of corn.
10. The food processing system according to clause 9, wherein the robot is configured move each ear of corn onto the processing conveyor such that a tip end of each ear of corn is oriented in a first direction and a tail end of each ear of corn is oriented in a second direction opposite the first direction.
11. The food processing system according to clause 10, wherein the processing includes a first blade configured to cut each ear of corn along the cutline plane determined for each ear of corn; and a second blade configured to cut off a tail end of each ear of corn.
12. The food processing system according to clause 11, wherein the first blade defines a blade cut plane; and
   wherein the robot is configured to move each ear of corn onto the processing conveyor such that the cutline plane for each ear aligns with the blade cut plane.
13. The food processing system according to clause 11, wherein during actuation of the blades the first blade is movable relative to the second blade which remains fixed in a position relative to the first blade.
14. A food processing system for individually processing ears of corn, the food processing system comprising:
   an imaging system having an x-ray for generating an image of each ear of corn;
   a control system configured to receive the image for each ear of corn from the imaging section and process the image to identify defects in the ear of corn and determine a product region of the ear of corn based on the identified defects;
   a conveyor configured to convey each ear of corn through the food processing system; and
   a robot configured to move each ear of corn from the conveyor into a processing system;
   wherein the processing system includes a processing conveyor onto which each ear of corn is received from the robot and a blade configured to cut each ear of corn based on the product region determined for each ear of corn such that each ear of corn is a processed ear of corn that corresponds to the product region.
15. The food processing system according to clause 14, wherein the control system is configured to determine an imaging position on the conveyor for each ear of corn based on the generated image for each ear of corn and further receive position data from the conveyor as the conveyor conveys the ears of corn in a downstream direction; and
   wherein the control system is configured to track position of each ear of corn along the conveyor based on the imaging position and the position data such that the robot can move each ear of corn from the conveyor to the processing conveyor.
16. The food processing system according to clause 14, wherein the processing system includes a first blade configured to cut each ear of corn along a cutline plane determined for each ear of corn by the control system, and a second blade configured to cut off a tail end of each ear of corn.
17. The food processing system according to clause 16, wherein the first blade defines a blade cut plane; and
   wherein the robot is configured to move each ear of corn onto the processing conveyor such that the cutline plane for each ear aligns with the blade cut plane.
18. The food processing system according to clause 16, wherein during actuation of the blades the first blade is movable relative to the second blade which remains fixed in a position relative to the first blade.
19. The food processing system according to clause 14, wherein the robot is configured move each ear of corn onto the processing conveyor such that a tip end of each ear of corn is oriented in a first direction and a tail end of each ear of corn is oriented in a second direction opposite the first direction.

## Claims

1. A food processing system for processing a food product, the food processing system comprising:
an imaging system for generating an image of the food product;
a control system configured to process the image of the food product and determine a product region related to the food product; and
a robot configured to move the food product into a processing section, wherein the processing section is configured to cut the food product and dispense a processed food product that corresponds to the product region.

2. The food processing system according to claim 1, further comprising a conveyor configured to convey the food product to the robot, and wherein the robot is configured to move the food product off the conveyor and further place the food product into the processing section.

3. The food processing system according to claim 2, wherein the processing section includes:
a processing conveyor onto which the food products are received; and
one or more blades configured to cut the food product and thereby form the processed food product as the processing conveyor conveys the food product past the one or more blades.

4. The food processing system according to claim 3, wherein the control system is configured to determine a cutline plane for the food product;
wherein one of the blades defines a blade cut plane; and
wherein the robot is configured to move the food product into the processing section such that the cutline plane aligns with the blade cut plane.

5. The food processing system according to claim 3 or 4, wherein the processing section includes two spaced apart blades that are configured to cut opposite ends from the food product.

6. The food processing system according to claim 5, wherein during actuation of the blades one of the blades is movable relative to the other blade which remains fixed in a cut position.

7. The food processing system according to claim 1:
wherein the imaging system has an x-ray for generating the image of the food product;
wherein the control system is configured to process the image of each food product and determine a cutline for the image and a corresponding cutline plane for each food product along which the food product is cut; and
the food processing system further includes a conveyor configured to convey each food product through the food processing system, and
- the processing section including:
a processing conveyor onto which the food product is received from the robot; and
a blade configured to cut the food product based on the respective cutline plane for the food product, and wherein the processing conveyor conveys the food product past the one or more blades such that each food product is a processed food product.

8. The food processing system according to claim 7, wherein the food product is one of a plurality of food products being ears of corn; wherein the robot is configured move each ear of corn onto the processing conveyor such that a tip end of each ear of corn is oriented in a first direction and a tail end of each ear of corn is oriented in a second direction opposite the first direction.

9. The food processing system according to claim 8, wherein the blade is a first blade configured to cut each ear of corn along the cutline plane determined for each ear of corn; and the food processing system further comprises a second blade configured to cut off a tail end of each ear of corn.

10. The food processing system according to claim 9, wherein the first blade defines a blade cut plane; and
wherein the robot is configured to move each ear of corn onto the processing conveyor such that the cutline plane for each ear aligns with the blade cut plane.

11. The food processing system according to claim 10, wherein during actuation of the blades the first blade is movable relative to the second blade which remains fixed in a position relative to the first blade.

12. The food processing system according to claim 1, wherein the food product is an ear of corn and wherein the food processing system is for processing a plurality of ears of corn;
wherein the imaging system has an x-ray for generating an image of each ear of corn in the plurality of corn; and
wherein the control system is configured to receive the image for each ear of corn from the imaging section and process the image to identify defects in the ear of corn and determine a product region of the ear of corn based on the identified defects;
wherein the food processing system further comprises:
a conveyor configured to convey each ear of corn through the food processing system; and
wherein the processing section includes a processing conveyor onto which each ear of corn is received from the robot and a blade configured to cut each ear of corn based on the product region determined for each ear of corn such that each ear of corn is a processed ear of corn that corresponds to the product region.

13. The food processing system according to claim 12, wherein the control system is configured to determine an imaging position on the conveyor for each ear of corn based on the generated image for each ear of corn and further receive position data from the conveyor as the conveyor conveys the ears of corn in a downstream direction; and
wherein the control system is configured to track position of each ear of corn along the conveyor based on the imaging position and the position data such that the robot can move each ear of corn from the conveyor to the processing conveyor.

14. Method for processing a food product, the method including the steps of:
- generating an image of a food product by means of an imaging system;
- processing the image of the food product and determining a product region related to the food product by means of a control system;
- moving the food product into a processing system by means of a robot; and
- by means of the processing system, cutting the food product and dispensing a processed food product that corresponds to the product region.

15. Method according to claim 14, wherein the step of generating an image includes the step of x-raying the food product.
